# EUROPEAN PATENT SPECIFICATION

(11) **EP 0 465 415 B1**
(45) Date of publication and mention of the grant of the patent: **22.01.1997**
(21) Application number: 91810484.5
(22) Date of filing: 20.06.1991
(51) Int. Cl.: C12N 5/12, C12P 21/08, G01N 33/569

(54) **Monoclonal antibodies to mycosphaerella species**
Monoklonale Antikörper gegen Mycosphaerella-Arten
Anticorps monoclonaux contre l'espèce mycosphaerella

(30) Priority: 29.06.1990 US 546341
(43) Date of publication of application: 08.01.1992
(73) Proprietor: CIBA-GEIGY AG, 4002 Basel (CH)
(72) Inventor: Petersen, Frank Peter, Burlington Township, New Jersey 08016 (US); Clymer, Mark Daniel, Norristown, Pennsylvania 19401 (US); Miller, Sally Ann, Pennsauken, New Jersey 08109 (US); Rittenburg, James Harley, Perkasie, Pennsylvania 18944 (US); Grothaus, Gary David, Burlington Township, New Jersey 08016 (US)

(56) References cited:
- EP-A- 0 246 615
- EP-A- 0 302 011
- JOURNAL OF GENERAL MICROBIOLOGY, vol. 135, no. 2, February 1989, Colchester (GB); M.M. Mac Donald et al.,pp. 375-383
- BOTANICA MARINA, vol. 21, 1978, Berlin (DE); N. Fries et al., pp. 409-411

## Description

The present invention relates to the field of diagnostic plant pathology. More specifically, the invention relates to monoclonal antibodies useful in the detection of species of the pathogenic fungal genus *Mycosphaerella*. One of the *Mycosphaerella* species to which monoclonal antibodies were made, namely *M. graminicola*, is more commonly known by the name of its anamorph (asexual stage) *Septoria tritici*.

### Fungal pathogens

Fungi as a group cause many plant diseases. For purposes of discussion the fungi can be classified as belonging to one of three major taxonomic classes: Basidiomycetes, Phycomycetes, or Ascomycetes.

### Basidiomycetes

Members of this class are identified by the presence of a sexual-spore forming structure known as a basidium. Pathogenic forms include smuts, rusts and fleshy species such as mushrooms. Examples include wheat rust, white pine blister, cedar-apple rust, and smuts causing disease in corn, oats, barley, onions and wheat.

### Phycomycetes

Members of this class are considered to be more primitive than members of either the Ascomycetes or Basidiomycetes, their distinguishing morphological feature being the absence of mycelial crosswalls. Examples of disease caused by members of the class include the downy mildews of grape and other hosts, root rot and late blight of potato and tomato.

### Ascomycetes

Members of this class possess a specialized reproductive structure (an ascus) in which meiosis and sexual spore formation take place. Examples of the more common plant diseases in which Ascomycetes have been identified as the etiologic agent include: powdery mildews on cereals, fruits and many other crops; Dutch elm disease; ergot of grains; peach and plum brown rot; black spot of roses as well as apple scab.

### Mycosphaerella Pathogens

With respect to the present invention, members of the family *Dothidiaceae*, and particularly the genus *Mycosphaerella*, are of particular interest. The genus is comprised of over 1,000 species, many of which are plant pathogens of great economic importance. A recent compilation of plant pathogenic fungi in the U.S. lists 258 species of *Mycosphaerella* as plant pathogens [Farr, D.F., Bills, G.F., Chamuris, G.P. & Rossman, A. 1989, Fungi on Plant and Plant Products in the United States, APS Press, Minneapolis: 1252 pp.]). The fruiting body of *Mycosphaerella* is an ostiolate, perithecioid pseudothecium containing eight two-celled ascospores contained in the bitunicate ascus. In many cases the ascospores are the primary mechanism of survival of pathogenic *Mycosphaerella* species in the absence of a susceptible host plant or under unfavorable environmental conditions. Ascospores often also serve as the primary source of inoculum in a host crop. Many of the species of *Mycosphaerella* also produce asexual, conidial (anamorph) stages; conidia may be borne in pycnidia, in acervuli, or on free conidiophores. In many instances the conidial stage is most often found in nature. The conidial stage is usually classified separately from the sexual stage on the basis of morphology. Thus, species in the genus *Mycosphaerella* have conidial stages classified as a number of different genera of Imperfect Fungi. For example, the conidial stage of *M. graminicola* is *Septoria tritici*, while the conidial stage of another *Mycosphaerella* species, *M. fijiensis*, is *Paracercospora fijiensis*. Several other examples include: *M. musicola* (*Pseudocercospora musae*); *M. fragariae* (*Ramularia brunnea*); *M. pinodes (Ascochyta pinodes*)); *M.tabifca* (*Phoma betae*); *M.tassiana* (*Cladosporium herbarum*).

The system of classification of asexual stages of ascomycetous fungi is generally considered to be artificial but continues for practical reasons, since the sexual stages are often difficult to find in nature and may be difficult to induce in pure culture. However, the degree of relatedness of species to one another is reflected in the sexual classification; i.e. two species of *Mycosphaerella* are likely to be more closely related genetically to one another than two species of *Septoria*, which are named on the basis of morphological similarities of the asexual stage.

Pathogenic species of *Mycosphaerella* cause disease on above-ground parts of plants, most often the leaves. Symptoms are lesions that may encompass a large portion of leaf area in irregular or regular patterns, depending on the crop. Some of the most severe and economically damaging diseases worldwide are caused by *Mycosphaerella* species, and require the use of resistant varieties and/or fungicides for control. *Mycosphaerella fijienris* and *M. fijiensis* var. *difformis*, the causal agents of Black Sigatoka (Black Leaf Streak) disease are widespread throughout banana and plantain growing areas of Central America, causing significant economic consequences as a result of yield loss or expenditures for fungicidal control. *Mycosphaerella graminicola,* more commonly known by its asexual stage, *Septoria tritici,* causes significant yield losses in wheat worldwide unless controlled by fungicides or resistant varieties.

*Mycosphaerella* species may exhibit a latent period of several days to several weeks after infection, in which the pathogen grows in the tissue but symptoms are not produced. Fungicides are most effective in controlling these diseases if applied during the latent period, before significant damage occurs and secondary inocula are produced that will allow the disease to spread. However, routine preventative fungicidal treatments are not usually economically or environmentally justified, and a system that would permit very early detection of these pathogens, preferably during the latent phase of disease development, would be very useful in assuring that fungicides are used appropriately and provide maximal economic benefit. The present invention enables just such a system to be put into practice by providing monoclonal antibodies that are capable of detecting the presence of *Mycosphaerella* antigens in the early stages of infection before disease symptoms appear, thus allowing early diagnosis of the disease, and possible prevention of widespread losses to the affected crop.

A number of monoclonal antibodies have now been produced which are capable of identifying various plant pathogens. For example, U.S. Patent No. 4,845,197 describes monoclonal antibodies which are capable of diagnosing infections caused by fungi in the family Pythiac*e*ae. U.S. Patent No. 4,803,155 describes monoclonal antibodies which are specific for members of the fungal genus *Sclerotinia*. U.S. Patent No. 4,879,217 discloses monoclonal antibodies which are specific for species of the genus *Rhizoctonia.*

No previous reports of antibodies having specificity for *Mycosphaerella* species have been made. Disclosed herein, however, are monoclonal antibodies that react specifically with *M. fijiensis* or *M. graminicola* or which react with more than one *Mycosphaerella* species, but not with other genera; these monoclonals are particularly useful for specific detection of *Mycosphaerella* infections on banana and wheat in the early stages of infection before disease symptoms appear.

Thus, the present invention provides hybridomas that produce monoclonal antibodies that react specifically with one species of the genus *Mycosphaerella* and which are capable of detecting Mycosphaerella injections in the early stages of injection before disease symptoms appear. The invention further provides hybridomas that produce monoclonal antibodies that react specifically with more than one species of the genus Mycosphaerella and which are capable of detecting Mycosphaerella injections in the early stages of injection. In a preferred embodiment, these antibodies react with members of the species *Mycosphaerella fijiensis* and/or *Mycosphaerella graminicola.* This should also be noted that the monoclonal antibodies which react with *Mycosphaerella* are also capable of reacting with antigens from the species' respective asexual stages, e.g., *Septoria tritici (M. graminicola)* or *Paracercospora fijiensis (M. fijiensis);* and the stage names are used interchangeably throughout the text and claims. The invention also encompasses specific hybridomas, which have the characterising features of HB 10413 and 10414, which react with more than one species of *Mycosphaerella,* and of HB 10186 which reacts specifically with *Mycosphaerella graminicola,* as well as clones and subclones thereof.

By clones and subclones of hybridoma cell lines are meant hybridomas which result from repeated cloning from the initial clone and which still have the features of the initial clone which are essential to the invention.

Likewise embraced by the present invention are mutants of each of these cell lines, which arise spontaneously or else can be prepared artificially by means of known methods and which still have the characteristic properties of the starting material, that is to say they are still able to produce and, preferably, secrete into the surrounding medium the antibodies according to the invention.

Also embraced by the present invention are monoclonal antibodies or derivatives thereof, that react specifically with one species or with more than one species of the genus *Mycosphaerella* and which are capable of detecting Mycospharella injections in the early stages of injection before disease symptoms appear. In a preferred embodiment, these antibodies or derivatives thereof react with members of the species *Mycosphaerella fijiensis* or *Mycosphaerella graminicola.* This should also be noted that the monoclonal antibodies which react with *Mycosphaerella* are also capable of reacting with antigens from the species' respective asexual stages, e.g., *Septoria tritici* (M. *graminicola)* or *Paracercospora fijiensis* (*M. fijiensis*)

By derivatives of monoclonal antibodies are meant within the scope of the present invention, for example, antibody fragments which still have high specificity and affinity for members of the genus *Mycosphaerella,* furthermore radioactively labelled monoclonal antibodies which are labelled, for example, with radioactive iodine (¹²⁵I, ¹³¹I), carbon (¹⁴C), sulfur (³⁵S), tritium (³H) or the like, conjugates of monoclonal antibodies with biotin or avidin, with enzymes such as horseradish peroxidase, alkaline phosphatase, β-D-galactosidase, glucose oxidase, glucoamylase, carbonic anhydrase, acetylcholinesterase, lysozyme, malate dehydrogenase or glucose-6-phosphate dehydrogenase, furthermore conjugates of monoclonal antibodies with bioluminescent (for example luciferase), chemoluminescent (for example acridinium esters) or fluorescent (for example phycobiliproteins) agents. Likewise embraced by the present application are bispecific and so-called cross-linked antibodies. This list of examples of possible antibody derivatives merely serves to illustrate the present invention and is not intended to limit the subject-matter of the invention in any way.

The availability of these monoclonal antibodies provides a means for diagnosing *Mycosphaerella* infections in plant material. Thus, a method for diagnosing such infections is provided which comprises contacting a plant samples suspected of containing antigens of the pathogen of interest with an antibody according to the invention and observing the presence or absence of a reaction between the antibody and antigen present in the sample. In a preferred embodiment, the assay is conducted as a sandwich, or double antibody assay: a first antibody that reacts with- the pathogen of interest is contacted with the plant sample, and then a second antibody which also reacts with the pathogen of interest is added, to form an antibody-antigen-antibody complex, if the antigen is present in the sample, wherein at least one of the antibodies is a monoclonal antibody according to the invention.

Also provided in this regard are compositions for detection of *Mycosphaerella* in form of a diagnostic kit which comprise an antibody, preferably immobilized, which reacts with the species of interest, and a detectably labelled antibody which reacts with the species of interest. At least one of the antibodies should be a monoclonal antibody of the present invention, and in certain embodiments, both antibodies may be monoclonals of the present invention.

The invention further provides a method for making a hybridoma cell line that produces monoclonal antibodies according to the invention and a method of obtaining monoclonal antibodies therefrom.

The monoclonal antibodies according to the invention are prepared using methods known *per se,* which are based essentially on the methods developed by Köhler and Milstein (1975). The said methods for producing the monoclonal antibodies according to the invention are also comprised by the present invention.

The antibodies produced are quite different than those recovered from antiserum from conventionally immunized animals. Each hybrid cell line synthesizes a homogeneous immunoglobulin that represents but one of the myriad of types of antibodies that an animal can synthesize in response to an antigen *in vivo*. Since each immunoglobulin-producing clone is characterized by the single type of antibody it produces, the term monoclonal antibody has been adopted.

The advantages of monoclonal antibodies are numerous:
a) monoclonal antibodies can be obtained in large supply and in high purity;
b) the preparation of monoclonal antibodies is homogeneous with respect to antigen reactivity and remains so over time;
c) hybridomas producing monoclonal antibodies can be stored for years and decades without losing their specific properties, i.e. the production of specific monoclonal antibodies, thereby;
d) monoclonal antibodies are more suitable for use as standard reagents than are polyclonal antisera, because the latter are adversely affected by a wide range of variation in respect of, for example;
α) the taking of blood from immunized animals for obtaining the antiserum,
β) a constant availability of material for additional immunizations,
γ) the limited lifespan of the donor animals.

The principle of hybridom/monoclonal technology is predicated on the observation that when two somatic cells are fused the resultant hybrid displays characteristics of both of the parent cell types. In the case of monoclonal antibody production, the ability to synthesize the particular antibody is derived from an immunocompetent cell (usually a spleen cell or a peripheral blood lymphocyte) taken from an immunized donor animal, whereas the ability to continuously divide in cell culture is contributed by the other fusion partner, a tumor cell line (often a myeloma). Early fusions were complicated by the fact that myeloma cell line also produced a monoclonal antibody; thus the hybrid often produced tow types of monoclonal antibody, one of myeloma origin and the other directed by the genetic information of the immunocompetent cell. Subsequently, tumor cell lines incapable of producing their own monoclonal have been used, e.g., SP2/0-Ag14 or X63-Ag8.653, thereby simplifying the analysis of the resultant fusion products.

Another technical consideration involves the rationale for selecting the successful fusion events (hybrid cells) from the two types of parental cells. Routinely a million or more cells of each type are used in the fusion protocol, and since fusion does not occur with 100 % frequency, the job of trying to recover fusion products from the high background of unfused or self-fused parents can be formidable. As mentioned hybridomas are formed by the fusion of short-lived antibody producing (spleen) cells and long-lived myeloma cells. The desired result is a long-lived cell line which produces antibody. Since the spleen cells have a finite life span in culture one can simply wait an appropriate period for all the nonfused or self-fused spleen cells to die; however one must still recover from the resultant population the long-lived antibody producing cells from the long-lived antibody non-producing cells. A popular means for selection hybrid cells is the so-called HAT-selection system. This system involves the use of the enzyme hypoxanthine-guanine-phosphoribosyl transferase (HGPRT). This enzyme functions in the purine salvage pathway in mammalian cells. These cells are also capable of synthesizing purines *de novo*. Under most conditions, both pathways probably operate to a certain extent. If a cell lacks HGPRT, the salvage pathway is blocked and purines must be manufactured from non-purine materials.

The chemical 8-azaguanine is an antimetabolite which is capable of masquerading as the purine guanine and replacing it in some of its normal reactions. Azaguanine is incorporated into DNA, interfering with the normal growth pattern and leading to cell death. Since azaguanine must be salvaged, any cell which lacks HGPRT activity cannot utilize azaguanine and will grow in its presence.

A selective system which operates on the same enzyme but in the opposite sense in that HGPRT positive cells are selected is described by J.W. Littlefield (1964). It is called HAT and contains hypoxanthine, aminopterin and thymidine (HAT medium). Aminopterin is an antimetabolite that prevents *de novo* purine synthesis and methylation of deoxyuridylate to form thymidylate. Hypoxanthine can seme as a salvagable purine in the event that aminopterin blocks *de novo* purine biosynthesis while thymidine bypasses the necessity for the methylation of thymidylate. Thus, in the presence of aminopterin, any cell with posibve HGPRT activity will proliferate while cells with negative HGPRT activity will die.

An alternate to the HAT system is the use of HMT medium in place of HAT. HMT employs amethopterin (methotrexate) in place of aminopterin. This method operates on the same principles, but the HMT medium is somewhat less toxic to the growing hybridomas, and therefore the cells can be left on the medium for a longer period of time.

In the hybrid system used for selection in accordance with the present examples, the myeloma cells are resistant to azaguanine and susceptible to amethopterin, that is, they are HGPRT negative. Thus, they will die in the presence of amethopterin. The antibody producing cells are HGPRT positive. By fusing the cells and growing them in HMT medium without azaguanine (HT medium), the successfully fused cells are selected because the myeloma cells which constitute the proliferating line can only grow where HGPRT activity is present and this activity must be supplied by the HGPRT positive cell line. The antibody producing HGPRT positive cell line are not killed in this medium. They will live for a time but will not proliferate.

Thus, by fusing the cells in a HAT or HMT medium, systems are produced in which the myeloma cells and antibody producing cells can grow long enough to produce hybrid cells but in which only the hybrid cells can survive and proliferate. After selection each hybridoma clone is then screened for the ability to produce the particular antibody of interest.

In a preferred embodiment of the instant invention *Mycosphaerella fijiensis* is used for the preparation of fungal extracts useful in the production of monoclonal antibodies. In particular, chopped mycelia (or a mixture of chopped mycelia and conidia) of a *Mycosphaerella* species can be employed as the antigen source. Methods of preparation of such extracts are discussed in detail in Example 1.1.

Spore extracts are also capable of yielding antigens which induce antibodies that react with one or more species of *Mycosphaerella*. Such extracts can be made from *Mycosphaerella graminicola*, either germinated or ungerminated spores, although germinated spores are preferred. The details of the extraction procedure are provided in Example 1.2.

Distinction between the antibodies so produced is made by a proper screening procedure, also outlined in detail below. To complete the formulation for inoculation, the antigen extract preferably is combined with an appropriate adjuvant. The adjuvants may be any which are commonly used in the art for this purpose; however, good results have been achieved using Freund's complete adjuvant for the first injection and Freund's incomplete adjuvant for any subsequent injections.

The antibodies described in the present examples were made using a suitable host organism such as mice as the antibody source. However, the identity of the experimental animal is not critical, and may be selected from any of the experimental animals commonly used for this purpose, such as rats, rabbits or goats.

The program for inoculation is not critical and may be any normally used for this purpose in the art. Such procedures are described, for example, in Goding, Monoclonal Antibodies: Principles and Pratice, Academic Press, New York, 1983.

A useful program is one in which a first immunization is given intraperitoneally, of the antigen combined with an appropriate adjuvant. Booster injections can then be administered at two week intervals. Two or more boosters may be given. The animals are tail bled to determine if the serum contains antibodies specific to the antigen of interest. The animal is subsequently sacrificed and the spleen removed to provide a source of lymphocytes.

Fusion procedures for creation of hybridomas are well known in the art, and any of the known procedures are useful for the production of hybridomas producing *Mycosphaerella*-specific monoclonals according to the invention. The basic procedure used in the present examples is a modification of that developed by Köhler and Milstein (1975) and Hammerling (1977).

To summarize briefly one procedure which has proven effective, spleen cells (or alternately, peripheral blood lymphocytes) are isolated from the immunized animal, and the number of cells counted. T cells used as a feeder layer at a concentration of 10 million cells/10 ml of medium have been employed successfully. An appropriate immortal cell line, preferably a myeloma cell line is selected and added to the lymphocytes in a ratio of about 4:1 lymphocytes:myeloma. A useful cell line is a cell line selected from the group consisting of the NS-1, SP2/0-Ag14 (Shulman *et al,* 1978) or X63-Ag8.653 myeloma cell line.

A preferred fusion medium is a buffer solution which contains one of the fusion promoters customarily used for fusing cells, such as, for example Sendai viruses or other paramyxoviruses, where appropriate in UV-inactivated form, calcium ions, surface-active lipids such as, for example, lysolecithin or polyethylene glycol. It is particularly preferred within the scope of this invention to use polyethylene glycol (PEG), preferably polyethylene glycol (PEG) with an average MW of 600 to 6000, especially of 1500, and in a concentration of 30 % to 60 %. A PEG concentration of 40 % - 50 % is particularly preferred. The optimal fusion temperature is between 18°C and 39°C. Room temperature is particularly preferred.

After addition of polyethylene glycol to the combined cells, the reaction mixture is diluted slowly with DMEM. After centrifugation, prewarmed HMT medium is added to the pellet which is resuspended. The sample is further diluted with HMT medium (see Example 1 for composition) and small samples distributed to the wells of a microtiter plate. The plates are then placed in a CO₂ incubation, preferably in a 9 % CO₂ incubation with at least 95 % humidity. Cells are re-fed with HMT medium after about 5-7 days. Clusters of hybridoma cells begin to appear after about 5-7 days. Further feedings are made with HT medium.

The above briefly summarized procedure for preparing a hybridoma cell line according to the invention just represents a preferred embodiment of the present invention but may not be construed as being limiting in any way.

The hybridoma cell clones according to the invention prepared as described are screened for the production of suitable monoclonal antibodies preferably using one of the immunoassays conventionally used for this purpose, such as, for example, an enzyme-coupled immunoassay or a radioimmunoassay.

In particular, those hybridomas producing antibodies to *Mycosphaerella* are identified using prepared fungal material of the particular species of interest, for example, either mycelia, conidia, or germinated spores in an ELISA format. In particular, mycelial material is a useful source from *M. fijiensis*, while germinated conidia are useful material from *M. graminicola*. Those wells demonstrating positive responses in the ELISA are subcloned to select pure strains of hybridoma cells. The subcloned lines are retested for specific antibody activity to fungal components.

In order to determine the degree of specificity of the selected antibodies, it is desirable to further screen them against a panel of fungal pathogens which may be either related or unrelated to the *Mycosphaerella.* For example, to obtain a *Mycosphaerella graminicola*-specific antibody the selected antibodies should be tested against other species of *Mycosphaerella,* as well as against more distantly related or unrelated species.

Examples of primary panels useful for the present purposes is presented in Tables 1 and 4. It is generally recommended to perform screenings in both a single and a double antibody format, as certain antibodies may be useful in one format, but not another.

A further embodiment of the present invention thus comprises a method for preparing a hybridoma cell line according to the invention, which method comprises:
(a) immunizing a donor animal with an extract of *Mycosphaerella;*
(b) isolating immunocompetent B cells from the immunized donor animal;
(c) fusing the B cells with cells of an immortal cell line;
(d) sceening fusion products and identifying those which produce antibodies having specificity for *Mycosphaerella;*
(e) cloning the fusion product isolated in step (d) to obtain a pure hybridoma cell line which produces a monoclonal antibody having specificity for *Mycosphaerella,* and being capable of detecting Mycosphaerella injections in the early stages of injection before disease symptoms appear.

Also comprised by the present invention are methods of preparing monoclonal antibodies according to the invention. In particular, these methods of preparing monoclonal antibodies are characterized in that the hybridoma cell lines according to the invention which have been characterized in detail previously, or else clones or subclones thereof, which synthesize and preferably secrete into the surrounding medium the antibodies according to the invention are cultivated *in vitro* or *in vivo* by means of known methods.

The *in vitro* cultivation of the hybridoma cells according to the invention is preferably carried out in suitable cultivation media, especially in the customarily used standardized culture media such as, for example, Dulbecco's modified Eagle medium (DMEM) or RPMI 1640 medium, each of which can be supplemented where appropriate by adding mammalian sera such as, for example, fetal calf serum, or by growth-promoting additives and trace elements.

The isolation of the monoclonal antibodies preferably starts with a precipitation of the immunoglobulin fraction from the particular supernatants of the hybridoma cultures, for example by using ammonium sulfate. This is followed by further working up and purification steps which are known to those skilled in this art and which include, for example, the use of chromatographic methods such as, for example, gel filtration, ion exchange chromatography, DEAE-cellulose chromatography, protein A or immunoaffinity chromatography.

However, it is also possible to obtain large amounts of the monoclonal antibodies according to the invention using *in vivo* methods.

Thus, for example, it is possible to inject antibody-producing hybridoma cell clones into suitable mammals, which induce the development of antibody-producing tumors in the treated animals. After a period of 1 to 3 weeks, the antibodies can be isolated from the body fluids of the animals treated in this way.

In a particular embodiment of the present invention, female Balb/c mice which have been pretreated, where appropriate, with a hydrocarbon such as, for example, pristane receive intraperitoneal injection of a hybridoma cell clone according to the invention. One to three weeks after the injection of the hybridoma cell clone the ascites fluid is collected and stored until worked up further.

The monoclonal antibodies are isolated in a manner exactly analogous to the previously described isolation from the supernatants of hybridomas cultivated *in vitro*.

A number of antibodies falling within the scope of one or another of the claims have been produced by the procedure as described herein. More than one antibody from those initially selected may have been produced by each fusion and screening; however, certain antibodies have been particularly noted, for their high level of specificity and/or their sensitivity in certain assay formats. Among the preferred antibodies for *Mycosphaerella* identification are those produced by hybridomas Mfl 4C6 and Mfdl 8E6. The hybridoma Mfl 4C6 produces an antibody having a high level of specificity for species of *Mycosphaerella*, with cross-reactivity observed only with the very distantly related species *Rhizopus stolonifer*. This property does not interfere with the antibodies' utility in detection of *Mycosphaerella*; moreover, MF14C6 is very sensitive in a double antibody format. The Mfdl 8E6 antibody shows good reactivity with various species of *Mycosphaerella*, and no cross reactivity with species of other genera. These antibodies are of the IgG1 immunoglobulin subclass.

With regard to *M. graminicola*, specifically the hybridoma Mg37B5 secretes antibodies of the subclass IgG3 which are highly specific and sensitive for *M. graminicola*. However, as can be seen from Table 1, other antibodies having these characteristics have also been produced.

Examples of hybridomas producing each of these types of antibodies have been deposited, under the terms of the Budapest Treaty, with the American Type Culture Collection (ATCC), Rockville, Maryland, USA, and have been given the following accession numbers:

| Hybridoma | Specificity | Accession No. |
|---|---|---|
| Mf14C6-E8 | Mycosphaerella species | HB 10413 |
| Mfd18E6 | Mycosphaerella species | HB 10414 |
| Mg37B5 | Mycosphaerella graminicola | HB 10186 |

It is to be understood that the above deposits are made for purposes of exemplification only, and the present claims are not to be construed as being limited thereby. One skilled in the art will readily recognize that similar antibodies can be prepared by repetition of the procedures described above.

The present invention further relates to the use of the antibodies according to the invention in one of the conventional immunoassays for detecting species of the pathogenic fungal genus *Mycosphaerella.*

As shown above, the pathogens in question are capable of causing serious damage to those plants which are infested by them, and early diagnosis is therefore highly desirable. The present antibodies now provide a method by which the pathogens can be detected in plant material before any visible symptoms of the disease appear on the plant.

The antibodies described above may be used as the basic reagents of a number of different immunoassays to determine the presence of a particular species in plant material. Generally speaking, the antibodies can be employed in any type of immunoassay, whether qualitative or quantitative. This includes both single site and two-site, or sandwich, assays of the non-competitive type, as well as in traditional competitive binding assays.

Particularly preferred, for ease of detection, and its quantitative nature, is the sandwich or double antibody assay, of which a number of variations exist, all of which are intended to be encompassed by the present invention.

For example, in a typical forward assay, unlabeled antibody is immobilized on a solid substrate and the sample to be tested brought into contact with the bound molecule. After a suitable period of incubation, for a period of time sufficient to allow formation of an antibody-antigen binary complex, a second antibody, labelled with a reporter molecule capable of inducing a detectable signal, is then added and incubated, allowing time sufficient for the formation of a ternary complex of antibody-antigen-labeled antibody. Any unreacted material is washed away, and the presence of the antigen is determined by observation of a signal, or may be quantitated by comparing with a control sample containing known amounts of antigen. Variations on the forward assay include the simultaneous assay, in which both sample and antibody are added simultaneously to the bound antibody, or a reverse assay in which the labelled antibody and sample to be tested are first combined, incubated and added to the unlabeled surface bound antibody. These techniques are well known to those skilled in the art, and the possibility of minor variations will be readily apparent. As used herein, "sandwich assay" is intended to encompass all variations on the basic two-site technique.

For the immunoassays of the present invention,the only limiting factor is that at least one antibody has the required specificity according to the invention. Thus, a number of possible combinations are possible. For example, one antibody may be polyclonal, and the other monoclonal. Alternately, one antibody may be a general antibody, which binds both the pathogen of interest and other fungi, while the second antibody is specific for the pathogen of interest. Also, both antibodies may be specific for the pathogen of interest.

As a more specific example, in a typical forward sandwich assay, a general *Mycosphaerella-binding* antibody is either covalently or passively bound to a solid surface.

Examples of solid support material suitable for the binding of the antigen or of the antibody are the plastic surface of a microtiter plate or of a test tube, the surface of beads made of polystyrene, polypropylene, polyvinyl chloride, glass or plastic or else the surface of strips of filter paper, dextran, cellulose or nitrocellulose, or similar materials. The latter are coated with one of the monoclonal antibodies according to the invention or with an antigen, it being possible for the binding to the support material to be brought about by simple adsorption or else, where appropriate, after preceding activation of the support material with, for example, glutaraldehyde or cyanogen bromide.

The preferred solid surface is usually glass or a polymer, the most commonly used polymers being cellulose, polyacrylamide, nylon, polystyrene, polyvinyl chloride or polypropylene.

The specific above-mentioned support materials can have a wide variety of designs and, depending on the particular intended specific purpose of use, very different types of shapes. The latter comprise, for example, dishes, spheres, plates, rods, cells, vials, tubes, fibres, nets, beads, discs or microplates, or any other surface suitable for conducting an immunoassay.

The binding processes are well-known in the art. Following binding, the solid phase-antibody complex is washed in preparation for the test sample. An aliquot of the plant extract to be tested is then added to the solid phase complex and incubated at a suitable temperature, preferably at 25°C, for a period of time sufficient to allow binding of any *Mycosphaerella* present to the antibody. The incubation period will vary, but will generally be in the range of about 2 minutes - 16 hours. Following the incubation period, the antibody-*Mycosphaerella* solid phase is washed and dried, incubated with a second antibody specific for *Mycosphaerella*. The second antibody is linked to a reporter molecule, the visible signal of which is used to indicate the binding of the second antibody to any antigen in the sample. By "reporter molecule", as used in the present specification and claims, is meant a molecule which by its chemical nature, provides an analytically detectable signal which allows the detection of antigen-bound antibody. Detection must be at least relatively quantifiable, to allow determination of the amount of antigen in the sample, this may be calculated in absolute terms, or may be done in comparison with a standard (or series of standards) containing a known normal level of antigen.

The most commonly used reporter molecules in this type of assay are either enzymes, fluorophores or radionuclide- containing molecules. In the case of an enzyme immunoassay an enzyme is conjugated to the second antibody, often by means of glutaraldehyde or periodate. As will be readily recognized, however, a wide variety of different conjugation techniques exist, which are well-known to the skilled artisan. Commonly used enzymes include horseradish peroxidase, glucose oxidase, β-D-galactosidase and alkaline phosphatase, among others. The substrates to be used with the specific enzymes are generally chosen for the production, upon hydrolysis by the corresponding enzyme, of a detectable color change. For example, p-nitrophenyl phosphate is suitable for use with alkaline phosphatase conjugates; for peroxidase conjugates, 1,2-phenylenediamine or tolidine are commonly used It is also possible to employ fluorogenic substrates, which yield a fluorescent product rather than the chromogenic substrates noted above. In all cases, the enzyme-labelled antigen-specific antibody is added to the first antibody-*Mycosphaerella* complex, allowed to bind to the complex, then he excess reagent is washed away. A solution containing the appropriate substrate is then added to the tertiary complex of antibody-antigen-labelled antibody. The substrate reacts with the enzyme linked to the second antibody, giving a qualitative visual signal, which may be further quantitated, usually spectrophotometrically, to give an evaluation of the amount of antigen which is present in the serum sample.

Alternately, fluorescent compounds, such as fluorescein and rhodamine, may be chemically coupled to antibodies without altering their binding capacity. When activated by illumination with light of a particular wavelength, the fluorochrome-labelled antibody absorbs the light energy, inducing a state of excitability in the molecule, followed by emission of the light at a characteristic longer wavelength. The emission appears as a characteristic color visually detectable with a light microscope. As in the EIA, the fluorescent labelled PLF-specific antibody is allowed to bind to the first antibody-ferritin complex. After washing of the unbound reagent, the remaining ternary complex is then exposed to light of the appropriate wavelength, and the fluorescence observed indicates the presence of interest.

Immunofluorescence and EIA techniques are both very well established in the art and are particularly preferred for the present method. However, other reporter molecules, such as radioisotopes, chemiluminescent of bioluminescent molecules may also be employed. It will be readily apparent to the skilled artisan how to vary the procedure to suit the required use.

The present invention thus further relates to means for the qualitative and quantitative determination of *Mycosphaerella* in the form of a test kit which can, besides the monoclonal antibodies according to the invention or their derivatives, contain, where appropriate in addition, other monoclonal or polyclonal antibodies, but especially labelled monoclonal or polyclonal antibodies, and further additives.

Particularly preferred within the scope of this invention are test kits which are based on one of the customarily used immunoassays selected from the group consisting of radioimmunoassay, enzyme-coupled immunoassay and chemiluminescence assay. Very particularly preferred test kits are those in which the detection of *Mycosphaerella* is based on a competitive immunoassay, but especially on an enzyme-coupled direct or indirect immunoassay (ELISA).

Test kits for a radioimmunological detection of *Mycosphaerella,* can contain, for example, the following constituents:
(a) a suitable support material which can be uncoated or else coated with one of the antibodies according to the invention or with an antigen conjugate;
(b) where appropriate freeze-dried or concentrated solutions of one of the antibodies according to the invention or of a radioactively labelled derivative thereof or radioactively labelled antigen or standardised solutions of the antigen;
(c) buffer solutions and
(d) where appropriate polypeptides, detergents and further additives which, for example, prevent non-specific adsorption and aggregate formation, and
(e) pipettes, reaction vessels, calibration plots, package inserts etc.

Test kits for the immunological detection of *Mycosphaerella* based on an enzyme-coupled immunoassay (ELISA) can contain, for example, the following constituents:
(a) a suitable support material which can be uncoated or else coated with one of the antibodies according to the invention or with an antigen conjugate;
(b) where appropriate freeze-dried or concentrated solutions of one of the antibodies according to the invention or of a second enzyme-labelled monoclonal or polyclonal antibody which is directed against the antigen to be determined or against an antibody recognising the antigen;
(c) enzyme substrates in solid or dissolved form;
(e) the antigen or standardised solutions of the antigen;
(f) buffer solutions;
(g) where appropriate polypeptides, detergents and further additives which, for example, prevent non-specific adsorption and aggregate formation, and
(h) pipettes, reaction vessels, calibration plots, colour tables, package inserts etc.

A test kit for the detection of *Mycosphaerella* which is based on a chemiluminescence test can contain, for example, the following constituents:
(a) a suitable support material which can be uncoated or else coated with one of the antibodies according to the invention or with an antigen conjugate;
(b) where appropriate freeze-dried or concentrated solutions of one of the antibodies according to the invention and of a second polyclonal antibody which is able to recognise the first antibody according to the invention and is linked to a chemiluminescent label;
(c) solutions containing a component which induces the emission of light, such as, for example, H₂O₂ and NaOH;
(d) buffer solutions;
(e) where appropriate polypeptides, detergents and further additives which prevent non-specific adsorption and aggregate formation and
(f) pipettes, reaction vessels, package inserts etc.

Support materials which can be used within the scope of the present invention primarily comprise insoluble polymeric materials selected from the group consisting of polystyrene, polyethylene, polypropylene, polyesters, polyacrylonitrile, polyvinyl chloride, polyacrylamide, nitrocellulose, crosslinked dextran, fluorinated resins, agarose, crosslinked agarose, polysaccharides etc. However, besides these, other materials are also conceivable, such as, for example, glass, metal, nylon-based net fabric etc.

The specific abovementioned support materials can have a wide variety of designs and, depending on the particular intended specific purpose of use, very different types of shapes. The latter comprise, for example, dishes, spheres, plates, rods, cells, vials, tubes, fibres, nets etc.

Frequently used for producing test kits are, for example, microtitre plates made from transparent plastic materials such as, for example, polyvinyl chloride or polystyrene, which can be uncoated or else coated with one of the antibodies according to the invention, with free antigen or with an antigen conjugate. Also used are beads, tubes or rods of polystyrene and polystyrene latex, in which case the surrounding latex material can be separated from the polystyrene particles by centrifugation.

Another component of the test kit according to the invention comprises labels or indicators which make it possible to detect the presence of a complex-forming reaction, but especially of an immunological reaction, which preferably results in an antigen-antibody complex or else in a ligand-receptor complex, in which case where appropriate quantitative information, besides qualitative, may be gained about the antigen to be detected. Suitable labels or indicators are both single atoms and molecules, which can be involved either directly or else indirectly in the generation of a detectable signal. These labels or indicators can be either linked directly to the antigen to be detected or to one of the monoclonal antibodies according to the invention, or else incorporated in the latter. However, they can also be in the form of single substances or of a component of a separate compound which is neither itself the antigen to be detected nor one of the monoclonal antibodies according to the invention but which in turn is able to react with the receptor molecule, for example in the form of a complex formation.

These separate compounds preferably take the form of a second antibody molecule which can be both monoclonal and polyclonal in origin, of a complement protein or fragments thereof, of S. *aureus* protein A etc. These separate compounds recognise and bind specifically to a receptor molecule such as, for example, the antigen to be detected or one of the monoclonal antibodies according to the invention, but preferably to a receptor molecule which is present in the form of a complex.
In many cases there is a need for further additional reagents which then lead to a detectable signal only on cooperation with the label. This particularly applies when enzymes are involved.

Labels or indicators which can be used within the scope of the present invention are very well known to the person skilled in the art of immunology and immunochemistry. They comprise, for example, radioactively labelled elements or substances, enzymes or chemiluminescent substances. The following list of possible labels or indicators is intended merely to these to illustrate the wide variety of substances and reagents which can be used by way of example but without thereby restricting the subject-matter of the invention in any way.
Examples of suitable labels or indicators may be found within the group of radioactive elements. Particularly preferred elements in this connection are those which either themselves emit γ rays, such as, for example, ¹²⁴I, ¹²⁵I, ¹²⁸I, ¹³²I,⁵¹Cr or else induce emission of these rays, such as, for example, ¹¹C, ¹⁸F, ¹³N. Likewise suitable are so-called β-emitters such as ¹¹¹In, ¹⁴C and ³H.

Other suitable labels comprise chemiluminescent substances, but especially fluorescent substances, which can be linked very simply by chemical means to the antigen or to an antibody without denaturing the latter. The resulting fluorochrome can easily be detected using fluorometric methods. Worthy of specific mention at this point are fluorochromes selected from the group consisting of fluorescein isocyanate, fluorescein isothiocyanate, 5-dimethylamino- 1-naphthalenesulfonyl chloride, tetramethylrhodamine isothiocyanate, lissamine, rhodamine 8200 sulfonyl chloride etc.

Further fluorescent agents and a description of analytical techniques are to be found in DeLuca, "Immunofluorescence Analysis", in: Antibody As a Tool, Marchalonis *et al*, John Wiley & Sons, Ltd., pp 189-231 (1982).

It is particularly preferred within the scope of this invention to use enzymes as labelling or indicator substances, such as, for example, horseradish peroxidase, alkaline phosphatase, β-D-galactosidase, glucose oxidase, glucoamylase, carbonic anhydrase, acetylcholinesterase, lysozyme, malate dehydrogenase, glucose-6-phosphate dehydrogenase etc. When enzymes are used as labelling substances it is necessary to add additional reagents which permit the formation of an immune complex to be followed via the enzyme activity and, where appropriate, a stop reagent with which the enzyme reaction can be stopped.

Particularly preferred in this connection are reagents which result in a colour reaction. In the case of horseradish peroxidase, an example which may be mentioned at this point is hydrogen peroxide which results, in combination with an additional oxidised dyestuff precursor such as, for example, diaminobenzidine or o-phenylenediamine, in a brown or yellow colour. When glucose oxidase is used as labelling substance it is possible to use, for example, 2,2′-azino-di-(3-ethyl-benzothiazoline-6-sulfonic acid) [ABTS] as substrate.

Thus the present invention further relates to the use of test kits which contain at least one of the monoclonal antibodies according to the invention as reagent, for the rapid and efficient, qualitative and/or quantitative detection of species of the pathogenic fungal genus *Mycosphaerella*.

### NON-LIMITING EXEMPLARY EMBODIMENTS

The following examples illustrate the methods of preparation of the hybridomas and antibodies of the present invention.

### Example 1: Antigen Preparation

### 1.1 Mycosphaerella fijiensis

Isolates of *Mycosphaerella fijiensis* are concurrently grown on V-8 agar plates. V-8 agar is prepared as follows:

| | |
|---|---|
| V-8 juice [vegetable juice: tomato, carrots, celery, spinach, beet, parsley, lettuce, (non-clarified); Campbell Soup Co., Camden, NJ 08103-1701] | 150 ml |
| calcium carbonate | 1.5 g |
| agar | 18.0 g |
| distilled water | 1.0 L |

If the isolate is already on a V-8 agar plate, 7-14 day old plates are flooded with 10 ml of sterile distilled water and conidia dislodged using a sterile cell scraper or glass rod. A small aliquot of the conidial suspension is aseptically removed and observed under the microscope for the presence of typical long and narrow *Cercospora*-like conidia. 0.1 ml of this suspension is placed onto V-8 agar plates and spread evenly over the surface using a sterile bent glass rod. The plates are incubated (parafilmed) under continuous light [GE F40; 201 White Fluorescent Light Bulbs, 24 hour photoperiod] at room temperature. Continuous transfers of the isolate may affect pathogenicity over time. Fresh isolates should be revived from liquid nitrogen every 3 months.

Isolates of *Mycosphaerella fijiensis* can be revived from liquid nitrogen by thawing the vial at room temperature and plating agar plugs onto concentrated V-8 agar plates. Plates are incubated at room temperature under continuous light. After visible growth appears on the agar plug (5-7 days), the plug is aseptically removed using sterile forceps and the plug is spread over the surface of V-8 agar plates.

Antigen can be produced using either conidia or chopped mycelium as the inoculant in broth cultures:

Conidial inoculum is prepared as described above using V-8 plates seeded with a conidial suspension. The inoculum plates are incubated at room temperature under continuous light for 7-14 days (10 days is optimal as reported in the literature). Plates are flooded with 10 ml of sterile distilled water and conidia dislodged as described above. 0.1 ml of the suspension is aseptically removed and using a hemacytometer, the number of conidia counted and the conidial concentration adjusted aseptically to 100,000 conidia/ml water. If done aseptically, 0.1 ml of the suspension can be used to seed additional V-8 plates. Previously, conidia numbers ranging from 500,000 to 900,000 could be harvested from a single agar plate.

Mycelial inoculum is prepared by inoculating 250 ml tissue culture flasks (Falcon-3024) containing 50 ml of sterile Phytone-Dextrose broth with either a conidial suspension (100,000 to 200,000 conidia) or mycelial chunks removed from an agar plate. Phytone-Dextrose broth is prepared as follows:

| | |
|---|---|
| Bacto-Soytone [Phytone; Difco Laboratories, Detroit, MI] | 10 g |
| Dextrose | 10 g |
| distilled water | 1 L |

The flasks are incubated upright for 3 days, then shaken vigorously and laid flat for approx. 5-7 days. After incubation, this suspension is treated physically to disrupt the mycelium, for example by transferring it aseptically to a sterile 50 ml Omnimixer chamber [stainless steel chamber (50 ml capacity) of an omnimixer disruptor; Omni International, Waterbury, Conn., USA]. This suspension is ground on high for 30 seconds to thoroughly chop the mycelium.

Aseptically, 200,000 conidia or 0.5 ml of chopped mycelial suspension are added to 50 ml of Phytone-Dextrose broth in a 250 ml flask and incubate with shaking in the water bath at 26°C and 80 rpm. The flasks are preferably incubated for 8 days.

Mycelium is harvested using coffee filters (6.4 x 8.3 cm) in a Buchner funnel under vacuum since *Mycosphaerella* grows in compact spheres in broth cultures which may pass through cheesecloth.

### 1.2: Mycosphaerella graminicola

Antigen extracts can be prepared from either germinated or ungerminated spores, as follows:

### 1.2.1: Ungerminated Spores

Conidia of an isolate of *S. tritici* are streaked out on a CDV8 plate (Czapek-Dox Broth, Difco Cat #0338-01-2, supplemented with "V-8" juice, Campbell's) using an inoculating loop. The plate is placed in 18°C incubation under near UV light. A 5-7 day old culture is flooded with sterile distilled water and the conidia gently dislodged by rubbing a sterile inoculating loop over the surface of the agar. The number of conidia/ml in the resulting suspension is determined using a hemacytometer (spore concentrations may be very high, a 1:50 dilution is recommended for a cloudy suspension). The spore suspension is aseptically diluted to a final concentration of 2 million conidia/ml with sterile distilled water.

Then 250 ml Ehrlenmeyer flasks containing 50 ml of yeast sucrose medium (YSM) [10 g yeast extract (Difco Laboratories); 10 g sucrose; 1 L water] with 1 ml of inoculum each (more flasks are necessary for a production batch) are aseptically inoculated. The YSM inoculated flasks are placed on a shaker [high speed; about 140 rpm] at 18°C with ambient light [fluorescent room lighting, approximately 10 hour photoperiod] and five of the flasks are harvested after 96 hours. The remaining flasks are used to prepare germinated spores, *infra*. The contents of the flasks are poured into a 250 ml centrifuge bottle and centrifuged at 3000 rpm for 5 minutes. The supernatant is poured off, and the pellet is washed once with distilled water, and recentrifuged. The pellet is resuspended in 50 ml of PBS and extracted in the Dyno-mill [Dyno-mill type KDL, cell disruptor. Impandes, Inc., Maywood, NJ 07067] for 15 minutes.

### 1.2.2: Germinated Spores

The five remaining flasks from the procedure described Above are harvested after 96 hours and allowed to germinate in water according to the following procedure.

The contents of each flask are aseptically transferred to a sterile Falcon tube and centrifuged at 3000 rpm for 5 min. The supernatant is poured off, washed once with sterile, distilled water, and recentrifuged. The pellet is resuspended completely in 50 ml sterile, distilled water. The suspension in each Falcon tube is transferred to a sterile, 250 ml Ehrlenmeyer flask. The inoculated flasks are placed on a shaker [high speed, about 140 rpm] at 18°C in ambient light for 72 hours. After 72 hours, the spores are harvested by pouring the contents of 5 flasks into one 250 ml centrifuge bottle (withdraw a 1 ml sample and determine % germination using a hemacytometer). The sample is centrifuged at 3000 rpm [GS-3 rotor, Sorvall centrifuge; 1521 g] for 5 minutes, the supernatant poured off, washed once with distilled water, and recentrifuged. The pellet is resuspended in 50 ml of PBS and extracted in the Dyno-mill [Dyno-Mill type KDL cell disruptor: Impandex, Inc., Maywood, NJ 07607] for 15 minutes.

### 1.3: Immunization and Fusion

The following description is for the *Mycosphaerella graminicola* immunization, but the *Mycosphaerella* immunization, using an *M. fijiensis* extract, is performed in an identical manner.

The first immunization is done intraperitoneally in a Balb/C mouse with 0.25 ml of Mg3 fungal extract [*Mycosphaerella graminicola* isolate 3, prepared as described in section 1.2.1] emulsified in 0.25 ml Freund's Complete Adjuvant. Booster injections are performed using the same immunogen in Freund's Incomplete Adjuvant at two week intervals. The fourth injection, third booster, is done with Mg3 fungal extract in incomplete adjuvant followed in two weeks with a booster of the same preparation. The mouse is sacrificed by cervical dislocation on the tenth day and the spleen excised for the extraction of lymphocytes. Prior to sacrifice, the mouse is tail bled and the serum tested for the presence of antibodies specific to the immunogen.

The mouse is sacrificed by cervical dislocation and the spleen surgically removed. The spleen is placed on an 80-mesh sterile screen in a sterile disposable petri dish, cut with a scalpel and massaged with a sterile plunger from a 3 cc syringe. During this procedure, the spleen is rinsed several times with DMEM. The cell suspension is transferred to a sterile 50 ml disposable centrifuge tube. The screen and the remaining tissue fragments are washed again with DMEM and the washings added to the tube. The cell suspension is spun down at 2000 rpm to 850 g [Beckman Table Top Centrifuge TH-4 rotor] for 10 minutes (centrifugations are all at room temperature). The supernatant is decanted and the pellet washed with 4 ml of red blood cell lysing solution (0.83 % NH₄Cl, 0.01 M KHCO₃, 0.1 mM EDTA) for 90 seconds at room temperature. The lysing reaction is halted by diluting the contents of the tube to 45 ml with DMEM. The contents are centrifuged again at 2000 rpm to 850 g [Beckman Table Top Centrifuge TH-4 rotor] for 10 minutes. The supernatant is decanted and the pellet is resuspended in 10 ml DMEM. A sample of the cell suspension is retained for cell counting. The total number of viable cells in the suspension is 2.985 x 10⁸ cells.

Myeloma cells [P3NS1/1-Ag4-1 (NS1) obtained from American Type Culture Collection, ATCC #TIB-18] are removed from culture medium by centrifugation in a 50 ml sterile centrifuge tube at 2000 rpm to 850 g [Beckman Table Top Centrifuge TH-4 rotor] for 10 minutes. The supernatant is decanted and the pellet resuspended in 10 ml DMEM for cell counting. 7.46 x 10⁷ myeloma cells are needed to give a 4:1 ratio of spleen cells to myeloma cells. The appropriate amount of myeloma cells are added to the spleen cell suspension. The combined cells are centrifuged at 2000 rpm to 850 g [Beckman Table Top Centrifuge TH-4 rotor] for 10 minutes. Following centrifugation, the supernatant is decanted.

1 ml PEG 1500 (Boehringer Mannheim Biochemicals, cat. #783-641) at room temperature is added to the pellet. The cells are very gently resuspended with a 2 ml pipet and an automatic pipettor. The cell suspension is diluted very slowly by the addition of 10 ml DMEM dropwise over 10 minutes with the gentle swirling of the tube to keep the cells in suspension. The suspension is then slowly diluted to 45 ml with DMEM and centrifuged 10 minutes at 1500 rpm to 500 g. The supernatant is decanted, the pellet washed gently with 10 ml DMEM and recentrifuged at 1500 rpm to 500 g for 10 minutes. The supernatant is decanted and prewarmed HMT medium is added to the tube. The pellet is resuspended, transferred to a disposable sterile culture flask, and diluted further to 80 ml with HMT medium. The final dilution is added to the inner 60 wells each of nine 96 well microtiter plates (Falcon, cat. #3870) using a 10 ml pipet, two drops per well. The outer row of each plate is then filled with DMEM+antibiotics. The microtiter plates are placed in a 9 % CO₂ incubator with at least 95 % humidity.

Cells are refed with HMT medium of the following composition after 5-7 days.

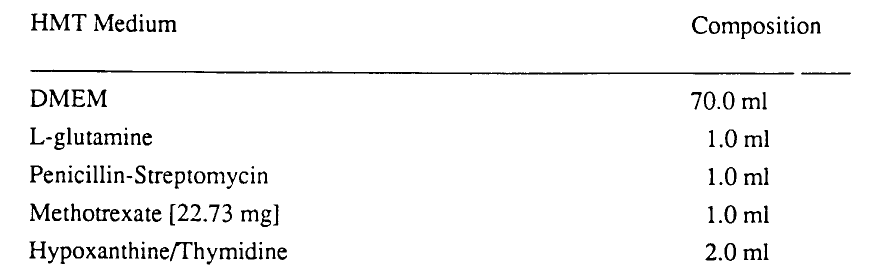

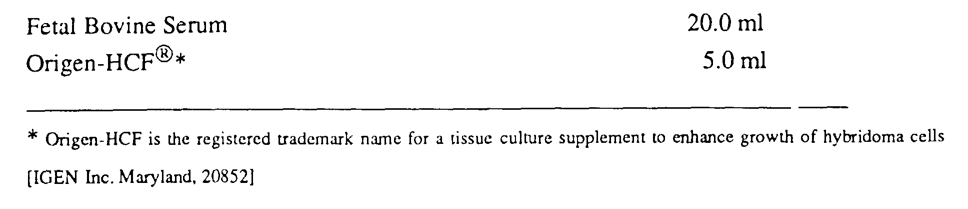

Clusters of hybridoma cells begin to appear after 5-7 days. Further feedings are made with HT medium, the same composition as HMT medium without methotrexate and Origen-HCF.

### 1.4: Screening for Hybridomas

### 1.4.1: Mycosphaerella graminicola

Hybridomas producing antibodies to fungal pathogens are identified using prepared extract of *Mycosphaerella graminicola* 30 % germinated spores (protein concentration 5 µg/ml in carbonate buffer) in an ELISA format. Those wells demonstrating positive response in ELISA's are subcloned so that pure strains of hybridoma cells are selected. The subcloning method involves diluting the cells from one well to <100 cells in 12 ml HT medium with 5% Origen-HCF. This dilute cell suspension is added to a microtiter plate, two drops per well. The wells of the subcloning plate are retested for specific antibody activity to fungal components. Positive wells are then transferred to larger culture vessels for mass culturing.

### ELISA -- Carbonate Procedure --

360 µl of carbonate buffer containing 5 µg/ml antigen is placed into each well of a microtiter plate (Nunc, cat. #468667) and incubated 3 hours at room temperature. The plates are washed 3 times with carbonate buffer. The wells are filled with blocking solution and incubated 30 minutes at room temperature. The remaining solution is shaken out, the plates patted on paper towels and dried overnight at 37°C in a mechanical convection incubator. The plates are then sealed in foil pouches containing desiccant bags and stored at 4°C.

Plates are removed from storage and allowed to warm to room temperature. 100 µl of supernatant sample is placed into each well and incubated 10 minutes on a microplate shaker at room temperature. The remaining solution is discarded and the plate is washed five times with wash buffer [20 mM Tris, 150 mM NaCl, 0.5 % Tween-80, 0.01 % Thimerosal (sodium ethyl mercurithiosalicylate; Fluka, Biochemika, # 71230), pH 7.8]. 100 µl of peroxidase-conjugated goat anti-mouse IgG [Kirkegaard and Perry Laboratories Inc. (KPL), Maryland 20879, cat. #14-18-02] diluted 1:1000 in 0.1 % BSA in PBS is added to each well. Plates are incubated 10 minutes on a shaker at room temperature. The remaining solution is discarded and the plates washed five times with wash buffer. 100 µl of ABTS substrate is placed in each well and the plates are incubated 10 minutes as before. The enzyme/ substrate reaction is stopped by adding 50 µl of 1.5 % NaF to each well and briefly mixing on the shaker. Absorbance is read at 405 nm.

### Required Solutions:

1. Carbonate Buffer: 0.1 M carbonate, pH 9.6, consists of 1.56 g/l Na₂CO₃ and 2.93 g/l NaHCO₃ in DI H₂O.
2. PBS: 2.19 g/l Na₂HPO₄, 0.56 g/l NaH₂PO₄, 8.76 g/l 1NaCl in DI H₂O, pH 7.2.
3. ABTS Stock: 15 mg/ml 2,2'-Azinobis(3-ethylbenzthiazoline sulfonic acid) in DI H₂O.
4. Citrate Buffer: 23 g/l Citric acid, pH 4.5, then add 0.5 ml 30 % H₂O₂.
5. ABTS Substrate: ABTS stock solution is diluted 1 ml with 24 ml citrate buffer.

All buffer solutions are made with anhydrous reagents when available and DI H₂O.

The results of a primary screening of number of antibodies against a panel of fungal pathogen antigens is shown in Table 1.

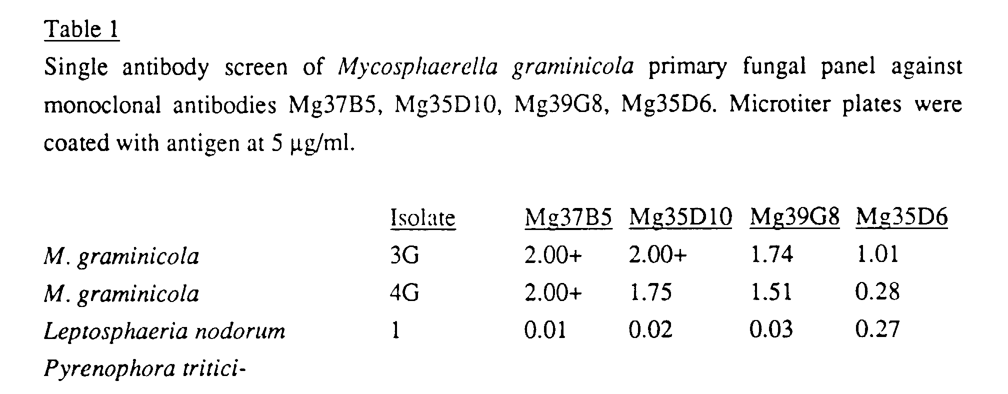

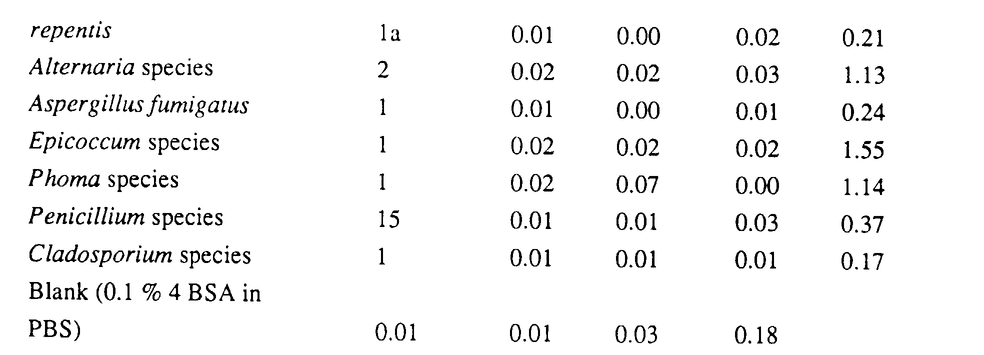

Single antibody screens were performed using antibody Mg37B5 against an expanded a panel of isolates of related fungal pathogens, as well as against several isolates of *Mycosphaerella graminicola.* The results are shown respectively, in Tables 2 and 3.

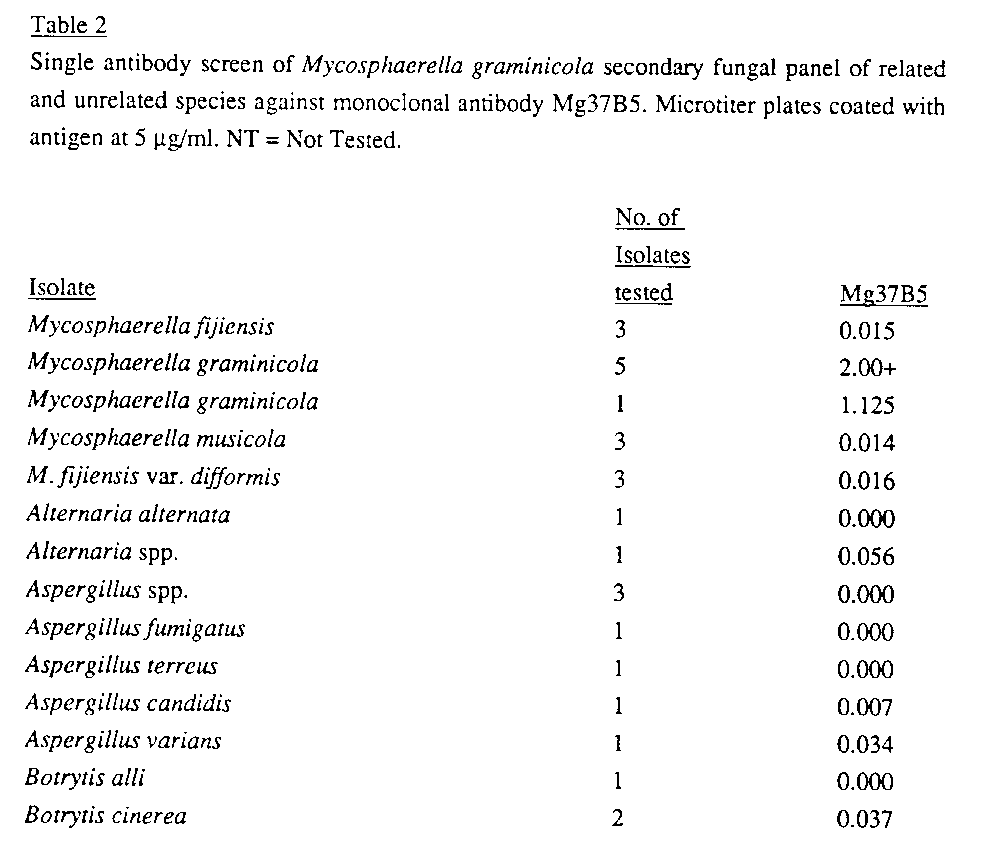

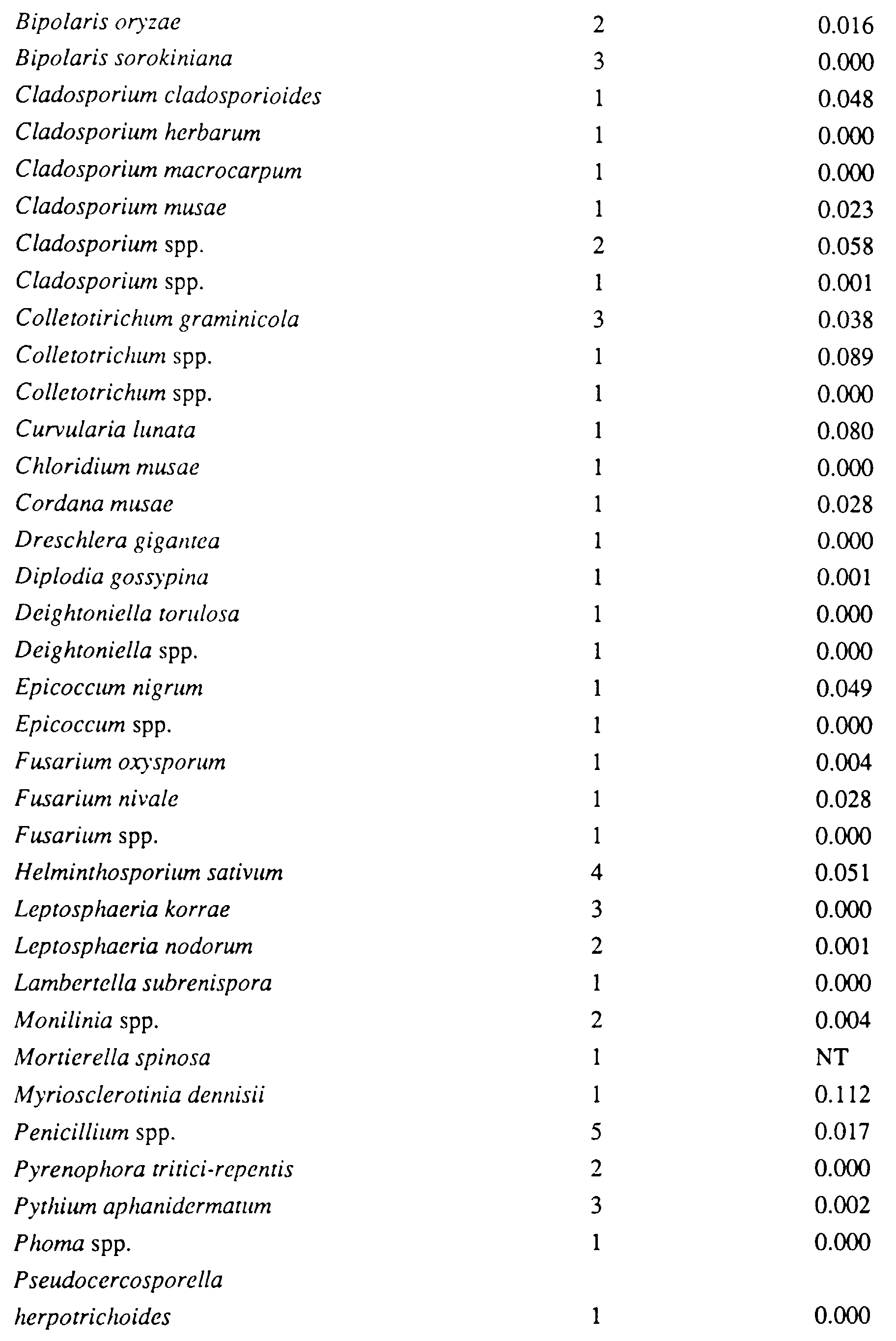

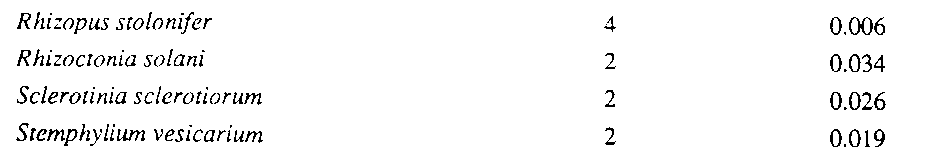

**Table 3**

| Single antibody screen of *Mycosphaerella graminicola* against monoclonal antibody Mg37B5. Microtiter plates were coated with antigen at 5 µg/ml. | | |
|---|---|---|
| Antigen | Batch | Monoclonal Antibody Mg37B5 |
| | | Absorbance (405 nm) |
| Mg 3 | 012G | 2.000+ |
| Mg 4 | 011G | 2.000+ |
| Mg 5 | 010G | 2.000+ |
| Mg 7 | 010G | 2.000+ |
| Mg 8 | 010G | 0.559 |
| Mg 10 | 010G | 2.000+ |
| Mg 15 | 010G | 1.125 |
| Mg 28 | 010G | 2.000+ |
| Mg 40 | 010G | 2.000+ |
| Mg 41 | 010G | 2.000+ |
| Mg 42 | 010G | 2.000+ |
| Mg 43 | 010G | 2.000+ |
| Mg 44 | 010G | 2.000+ |
| Mg 45 | 010G | 2.000+ |

Three cell lines from the *Mycosphaerella graminicola* fusion were tested against the fungal isolates of the primary cross reactivity panel for *Mycosphaerella fijiensis* in a single antibody format. As shown in Table 4, each of these antibodies (all IgG1) shows a strong cross-reactivity with *Mycosphaerella* species, as well as being capable of reacting with *Mycosphaerella graminicola.*

**Table 4**

| Single antibody screen of *Mycosphaerella fijiensis* primary fungal panel against monoclonal antibodies Mg39G8, Mg35D10, and Mg35D3. Microtiter plates were coated with antigen at 5 µg/ml. | | | | |
|---|---|---|---|---|
| | Isolate | Mg39G8 | Mg35D10 | Mg35D3 |
| *Mycosphaerella fijiensis* | 1 | 1.30 | 0.74 | 0.80 |
| *M. fijiensis* var. *difformis* | 1 | 0.07 | 0.27 | 0.37 |
| *M. fijiensis* var. *difformis* | 3 | 1.66 | 1.83 | 1.89 |
| *Mycosphaerella musicola* | 1 | 1.30 | 1.56 | 1.80 |
| *Mycosphaerella graminicola* | 3G | 1.55 | 1.33 | 1.61 |
| *Deightoniella torulosa* | 1 | 0.00 | 0.02 | 0.08 |
| *Cladosporium herbarum* | 1 | 0.00 | 0.03 | 0.06 |
| *Dreschlera gigantea* | 1 | 0.00 | 0.00 | 0.03 |
| *Cladosporium* | | | | |
| *cladosporioides* | 1 | 0.00 | 0.00 | 0.03 |
| *Cladosporium musae* | 1 | 0.01 | 0.03 | 0.08 |
| *Rhizopus stolonifer* | 2 | 0.00 | 0.00 | 0.03 |

The monoclonal antibody of hybridoma Mg37B5 and other monoclonals, was further tested in a double antibody format against an infected wheat, and other comparative material, as follows:

Monoclonal antibodies are purified from hybridoma supernatant samples by Protein A affinity chromatography. The eluant from the chromatography column is dialyzed against two changes of PBS, >100 volumes per dialysis cycle. The protein concentration of the antibody solution is calculated by dividing the absorbance of the solution at 280 nm by 1.4 to give the concentration in milligrams of protein per milliliter.

Microtiter plates are sensitized by first diluting the purified antibody solution in the appropriate sensitization buffer and then placing 100 µl in each well. The concentration of antibody following dilution is 5 µg/ml in each of three sensitization buffers; Carbonate Buffer, pH 9.6, PBS, pH 7.2 and 0.1 M Glycine-HCL, pH 4.5. The microtiter plates are incubated 3 hours at room temperature. The remaining solution is discarded and the wells are washed 3 times with the same buffer as that used for sensitization. The wells are filled with blocking solution and incubated 30 minutes at room temperature. The remaining solution is discarded, the plates blotted on paper towels and then dried at 37°C overnight in a mechanical convection incubator. The plates are then sealed in foil bags containing a small desiccant pouch and stored at 4°C.
Plates are removed from storage and allowed to equilibrate at room temperature. Antigen solutions are prepared by diluting fungal culture extracts in 0.1 % BSA/PBS or by grinding infected wheat leaves in the same buffer. 100 µl of the antigen solution is added to each well and the plates are incubated 10 minutes at room temperature on a microtiter plate shaker. The remaining solution is discarded and the plates are washed 5 times with wash buffer. 100 µl of peroxidase-conjugated sheep anti-Mg3G (0.5 µg/ml in 0.1 % BSA/PBS) is added to each well. The plates are incubated as before. The remaining conjugate solution is discarded and the plates washed 5 times with wash buffer. 100 µl of ABTS [2,2'-Azinobis (3-ethyl-benzthiazoline sulfonic acid)] substrate is added to each well and the plates are incubated as before. The color reaction is stopped after 10 minutes by adding 50 µl of 1.5 % NaF to each well and mixing briefly on the plate shaker. The absorbance of each well is read at 405 nm.

The results, which are shown in Table 5, indicate that the monoclonals of Mg37B5 are effective in detecting *Mycosphaerella graminicola* in infected plant material.

**Table 5**

| Double antibody test using supernatant of cell line Mg37B5 as the capture antibody (concentration of antibody 5 µg/ml, diluent carbonate buffer pH 9.6). Conjugate used was sheep anti-*Mycosphaerella graminicola.* The antigens tested were wheat infected with *Mycosphaerella graminicola,* healthy wheat, and Mg3G isolate. | |
|---|---|
| Antigen | Mg37B5 |
| *Mycosphaerella graminicola* infected wheat dilution 1:10 | 0.78 |
| | |
| Healthy wheat | 0.02 |
| | |
| *Mycosphaerella graminicola* isolate 3G 10 µg/ml | 2.0+ |
| | |
| 0.1 % Bovine Serum Albumin | 0.01 |

### Mycosphaerella-Fijiensis

The screening of hybridomas resulting from *Mycosphaerella fijiensis* fusions was performed in substantially the same manner as described for *Mycosphaerella graminicola,* supra. The results of the primary screening panel are shown in Table 6.

**Table 6**

| Single antibody screen of *Mycosphaerella fijiensis* primary fungal panel against monoclonal antibodies Mf14C6, Mf18E6, Mfd12C3, Mfd13F9, Mfd116D8, Mfd11B9, Mfd18C11 and Mfd17D10. Microtiter plates were coated with antigen at 5 µg/ml. NT = Not Tested. | | | | | | | | |
|---|---|---|---|---|---|---|---|---|
| | Mf14C6 | Mfd18E6 | Mfd12C3 | Mfd13F9 | Mfd16D8 | Mfd11B9 | Mfd18C11 | Mfd17D10 |
| *Mycosphaerella fijiensis* | 0.21 | 2.00+ | 2.0+ | 0.08 | 0.02 | 0.69 | 0.79 | 1.64 |
| *M. fijiensis* var. *difformis* | 0.12 | 2.00+ | 2.0+ | 0.49 | 0.82 | 1.65 | 0.61 | 1.83 |
| *M. fijiensis* var. *difformis* | 0.07 | 2.00+ | 2.0+ | 0.09 | 0.06 | 0.00 | 0.36 | 0.95 |
| *Mycosphaerella musicola* | 0.00 | 2.00+ | 2.0+ | 0.09 | 0.04 | 0.21 | 0.55 | 1.74 |
| *Mycosphaerella graminicola* | 0.00 | 1.05 | 0.82 | 0.09 | 0.05 | 0.01 | 0.28 | 0.11 |
| *Deightoniella torulosa* | 0.00 | 0.21 | 0.18 | 0.08 | 0.10 | 0.00 | 0.64 | 0.00 |
| *Cladosporium herbarum* | 0.00 | 0.00 | 0.02 | 0.00 | 0.01 | 0.00 | 0.12 | 0.27 |
| *Dreschlera gigantea* | 0.00 | 0.00 | 0.01 | 0.04 | 0.00 | 0.00 | 0.07 | 0.00 |
| *Cladosporium musae* | 0.00 | 0.00 | 0.08 | 0.06 | 0.02 | 0.00 | 0.03 | 0.00 |
| *Fusarium oxysporium* | 0.00 | 0.00 | 0.04 | 0.08 | 0.00 | 0.00 | 0.05 | 0.00 |
| *Rhizopus stolonifer* | 0.02 | 0.05 | 0.12 | 0.07 | NT | 0.00 | 0.60 | 0.00 |
| Blank | 0.00 | 0.00 | 0.06 | 0.09 | NT | 0.00 | 0.00 | 0.00 |

The results of a single antibody assay testing cross reactivity of several *Mycosphaerella* antibodies against a broad panel of related and unrelated isolates is provided in Tables 7 and 7a. The reactivity of these monoclonal antibodies with multiple isolates of *M. fijiensis* is shown in Tables 8 and 8a.

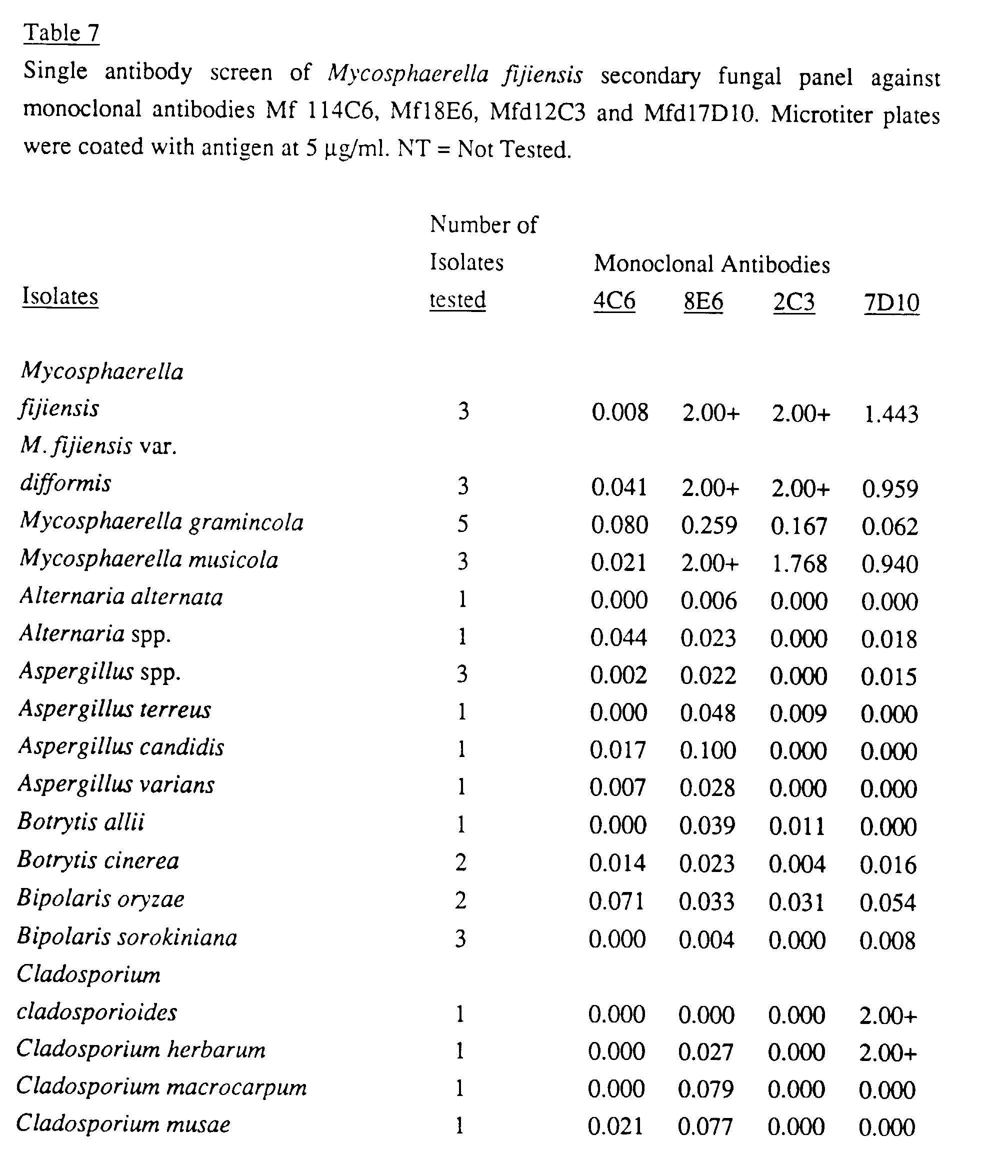

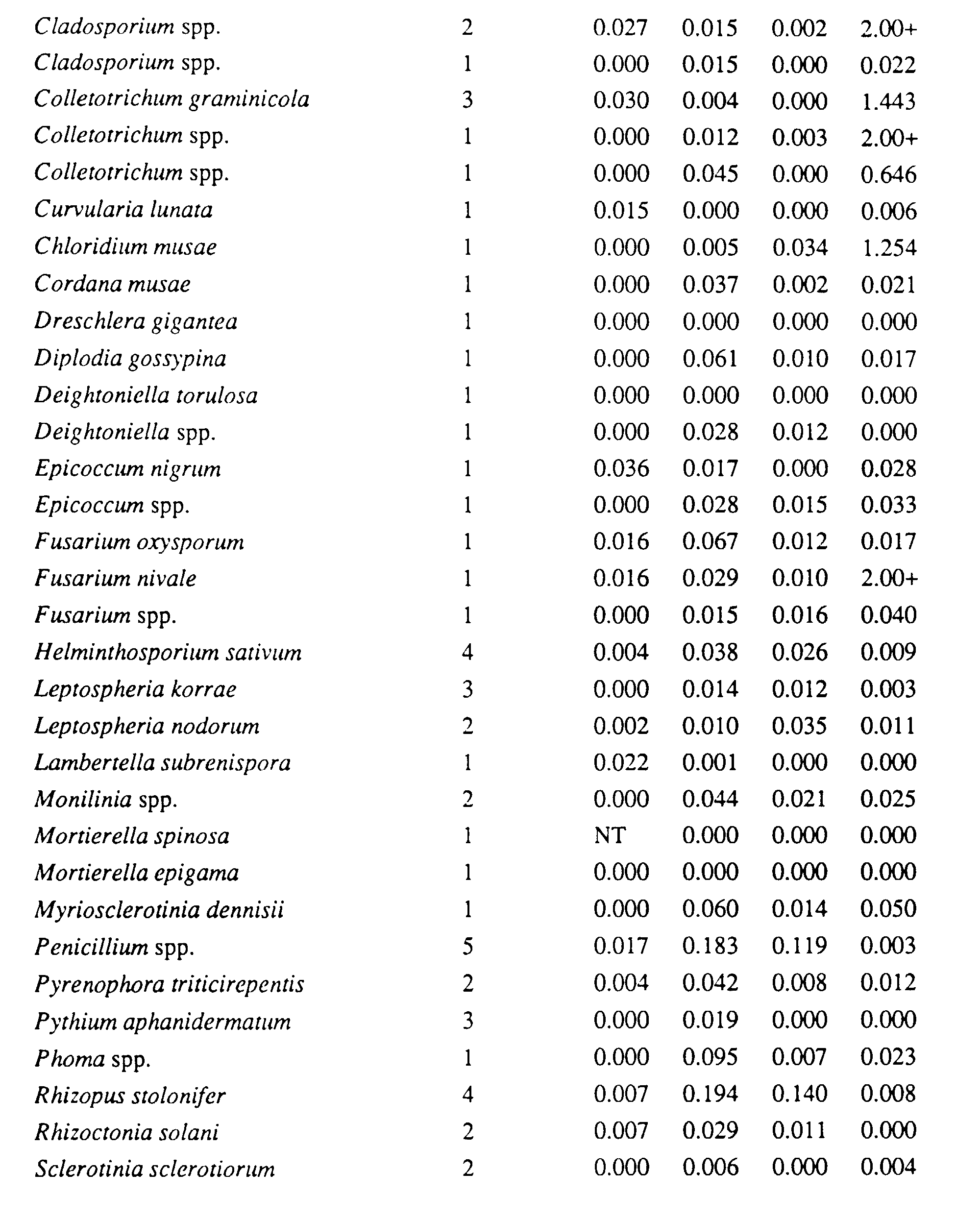

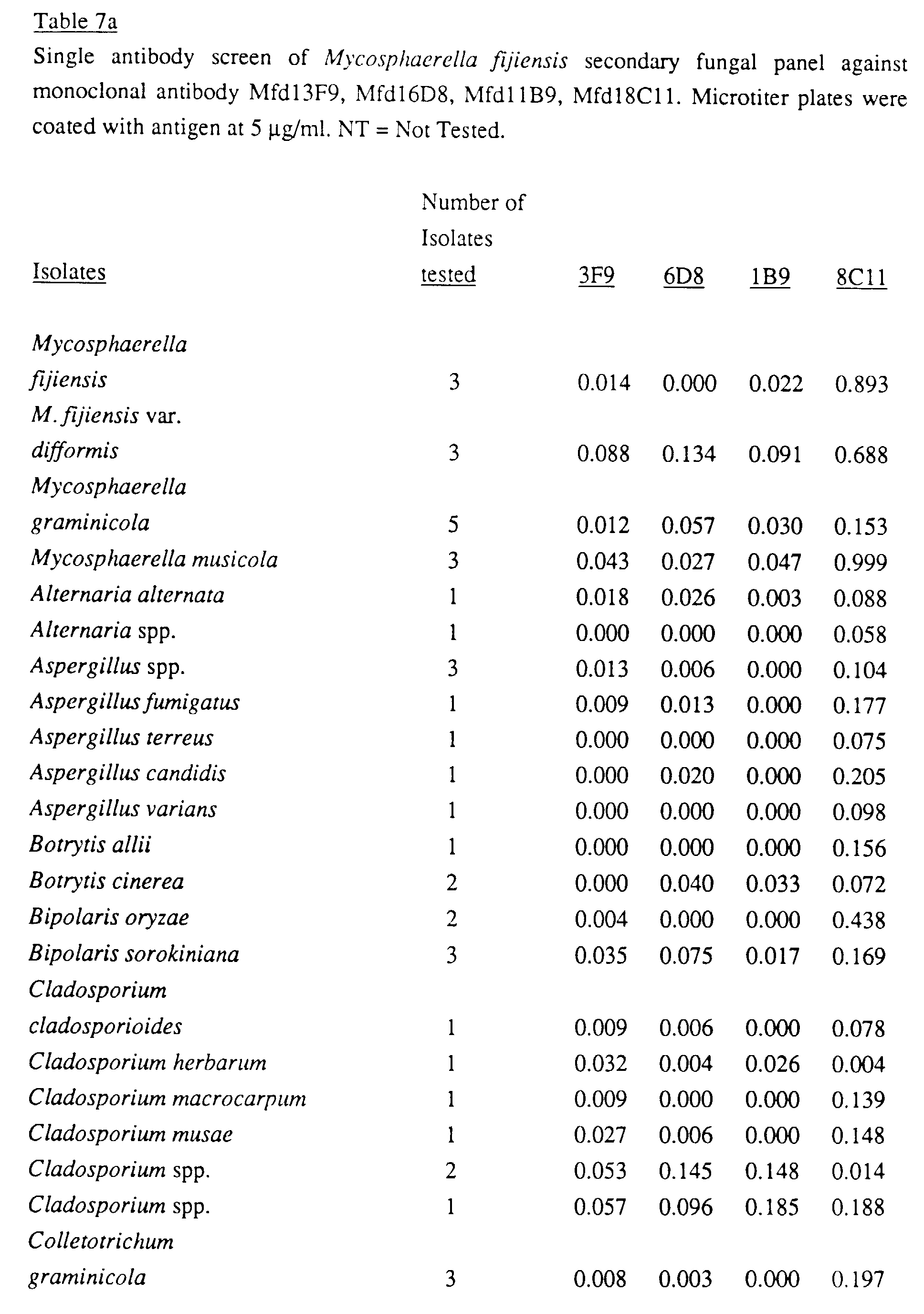

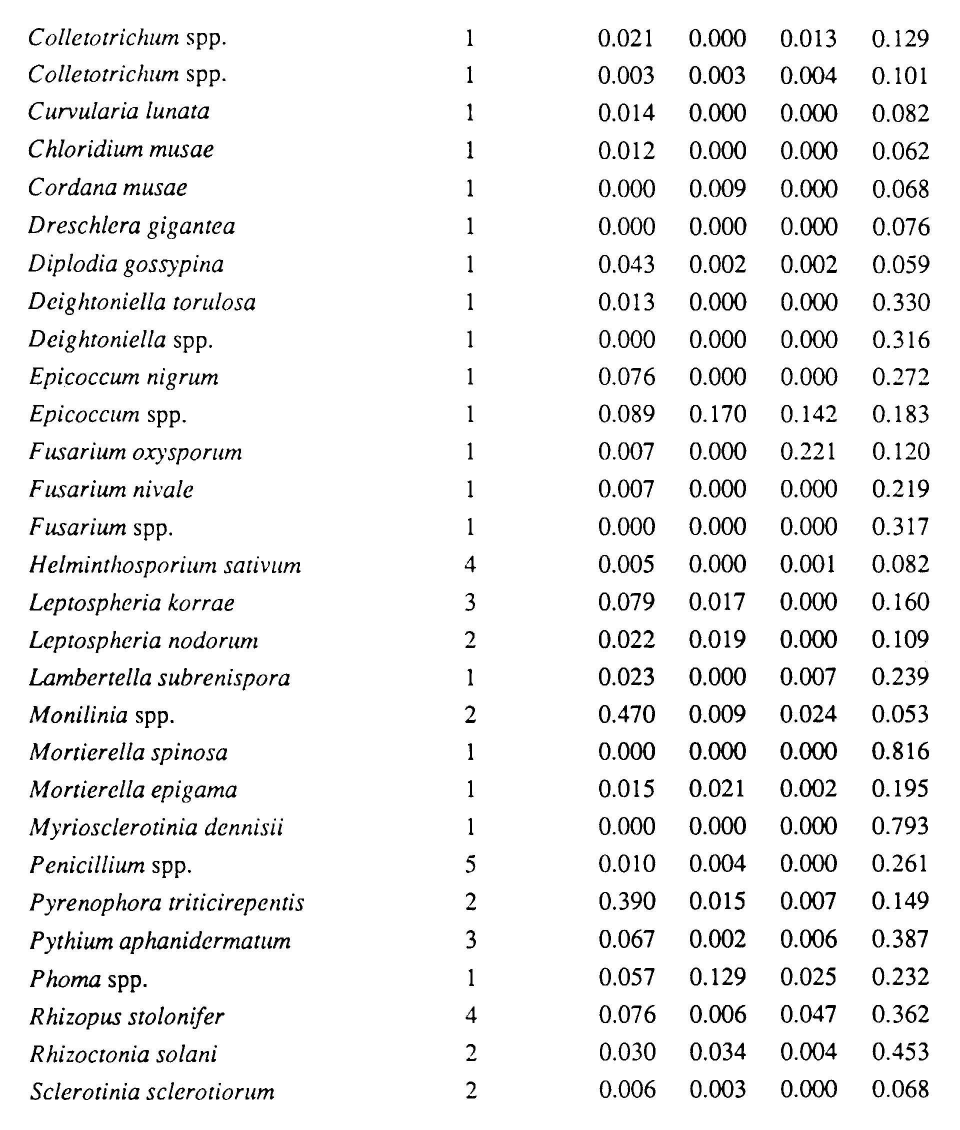

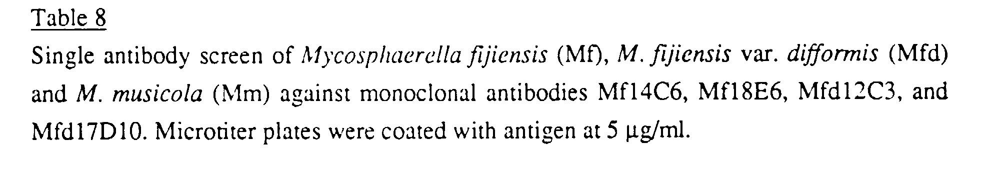

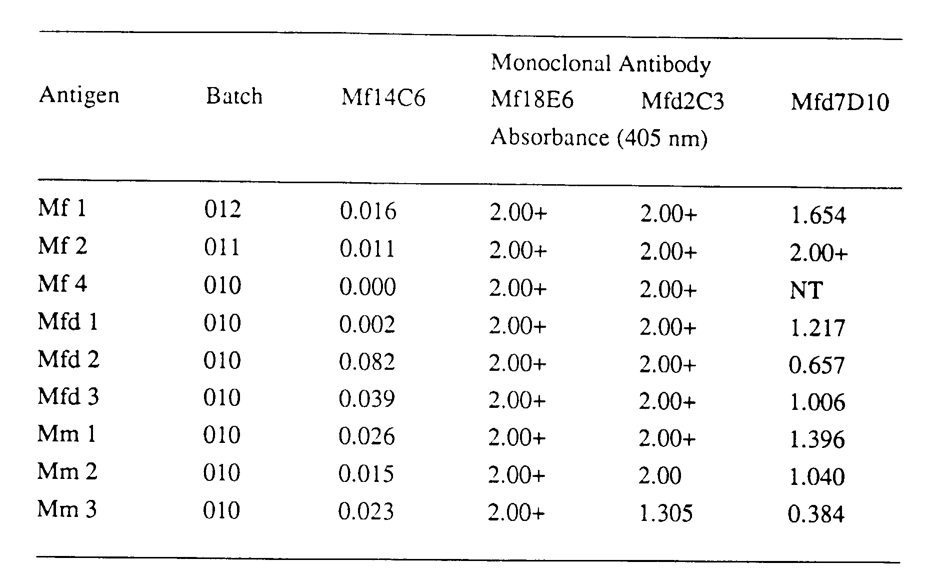

**Table 8a**

| Single antibody screen of *Mycosphaerella fijiensis* (Mf), *M. fijiensis* var. *difformis* (Mfd) and *M. musicola* (Mm) against monoclonal antibodies Mfd13F9, Mfd16D8, Mfd11B9, Mfd18C11. Microtiter plates were coated with antigen at 5 µg/ml. | | | | | |
|---|---|---|---|---|---|
| Antigen | Batch | Monoclonal Antibody | | | |
| | | Mfd 3F9 | Mfd 6D8 | Mfd 1B9 | Mfd 8C11 |
| | | Absorbance (405 nm) | | | |
| Mf 1 | 012 | 0.017 | 0.000 | 0.023 | 0.328 |
| Mf 2 | 011 | 0.000 | 0.000 | 0.048 | 0.350 |
| Mf 4 | 010 | 0.025 | 0.009 | 0.000 | 2.000 |
| Mfd 1 | 010 | 0.228 | 0.377 | 0.257 | 0.146 |
| Mfd 2 | 010 | 0.004 | 0.000 | 0.000 | 0.253 |
| Mfd 3 | 010 | 0.032 | 0.025 | 0.015 | 1.666 |
| Mm 1 | 010 | 0.000 | 0.000 | 0.000 | 2.00+ |
| Mm 2 | 010 | 0.021 | 0.007 | 0.000 | 0.370 |
| Mm 3 | 010 | 0.109 | 0.074 | 0.141 | 0.627 |

A double antibody screen of *Mycosphaerella fijiensis* monoclonal antibody Mf14C6 against a broad panel of related and unrelated fungal pathogens is shown in Table 9. This table illustrates both the high level of specificity of the Mf14C6 antibody and the greater sensitivity of the Mf14C6 in a double antibody versus a single antibody screen. Table 10 shows results of double antibody screen at Mf14C6 against a panel of *Mycosphaerella fijiensis* infected banana leaves, and pure culture of *Mycosphaerella fijiensis* as well as healthy banana leaves.

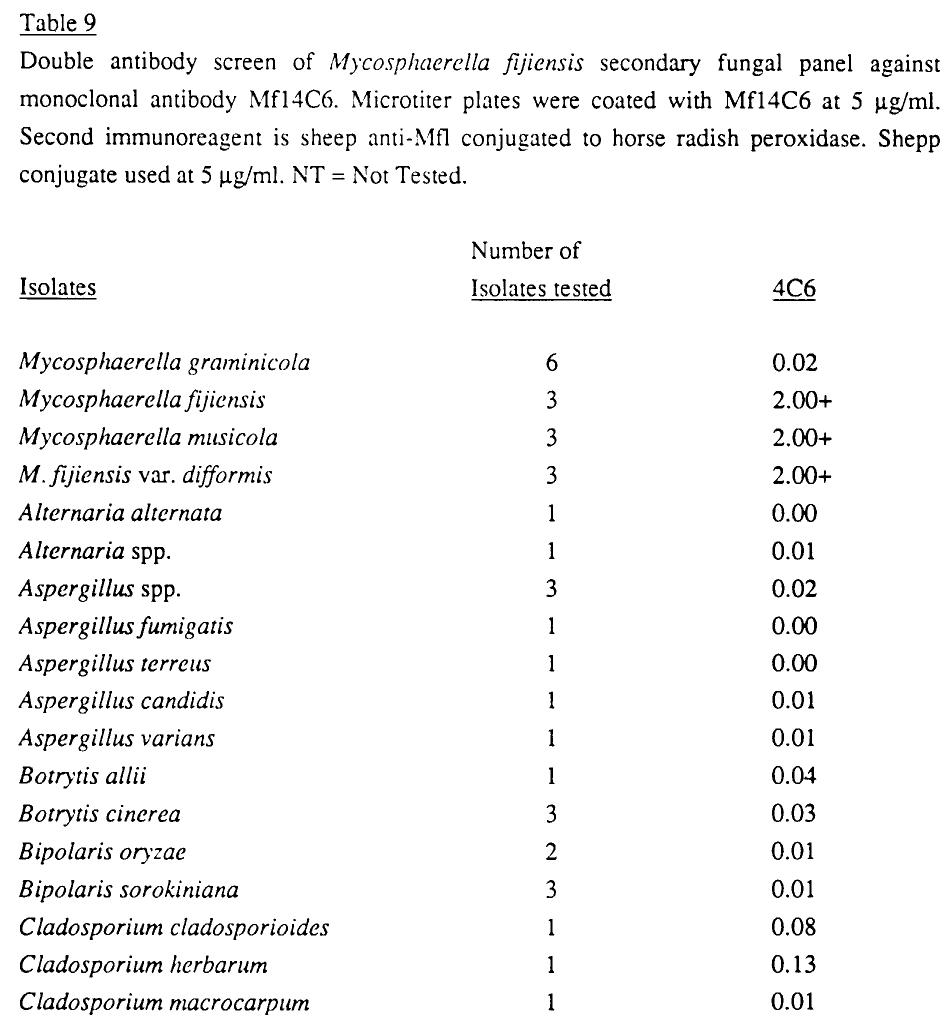

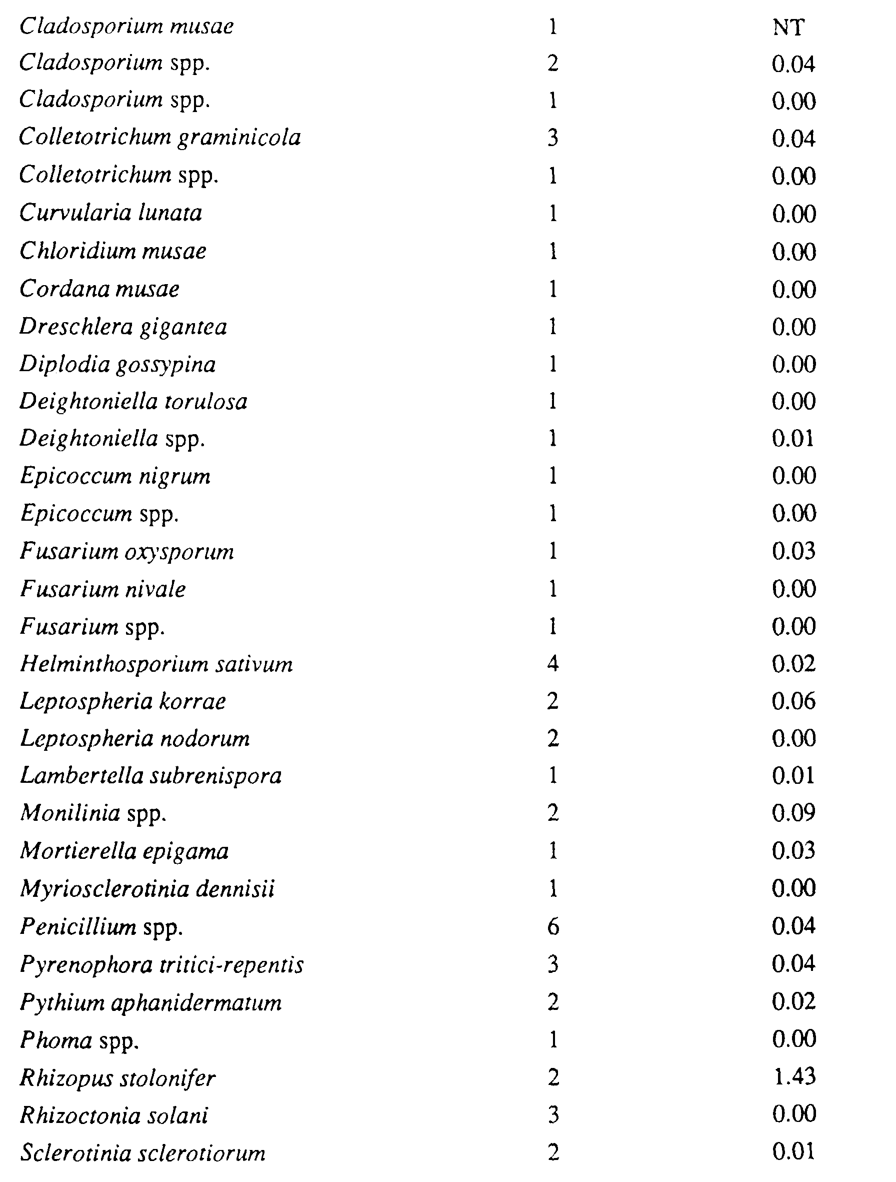

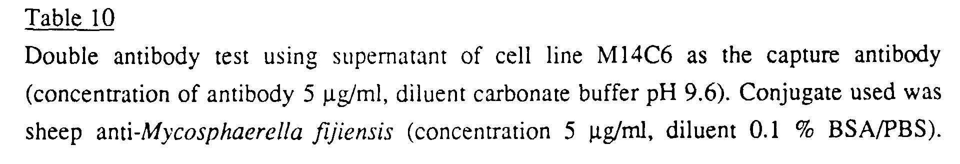

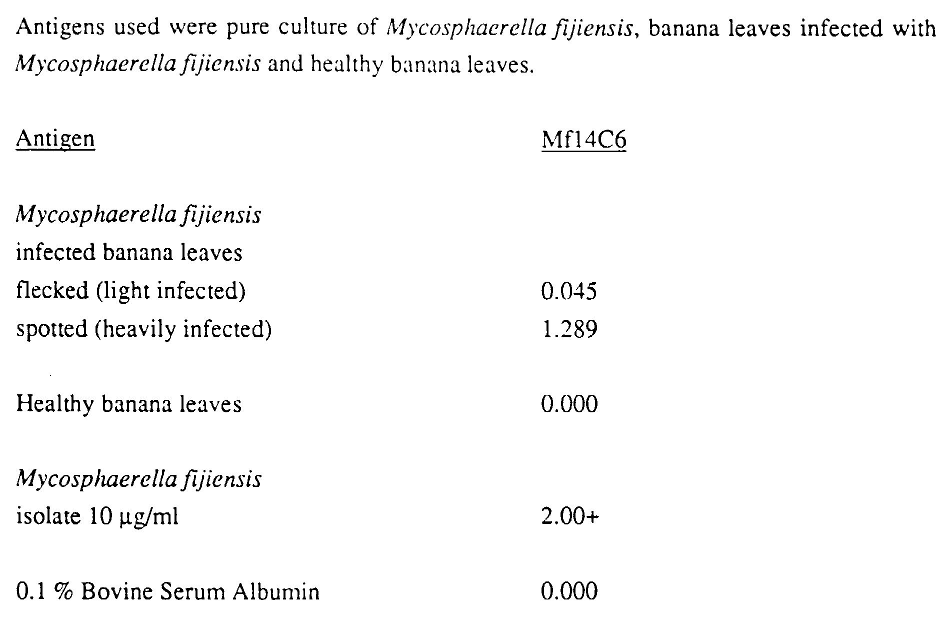

### BIBLIOGRAPHIE

**DeLuca**, "Immunofluorescence Analysis", in: Antibody As a Tool, Marchalonis *et al*, John Wiley & Sons, Ltd., pp 189-231 (1982)
**Farr**, DF *et al*, Fungi on Plant and Plant Products in the United States, APS Press, Minneapolis: 1252 pp. (1989).
**Goding**, Monoclonal Antibodies: Principles and Pratice, Academic Press, New York, 1983.
**Hammerling** (Eur. J. Immunol. 7:743, 1977).
**Köhler** and Milstein Nature, 256: 495-497, (1975).
**Littlefield** JW, Science, 145:709 ( 1964).
**Shulman** *et al*, Nature, 276: 269-270 (1978).

U. S. Patent No. 4,845,197
U.S. Patent No. 4,803,155
U.S. Patent No. 4,879,217

## Claims (Claims for the following Contracting State(s): AT, BE, CH, DE, DK, FR, GB, GR, IT, LI, LU, NL, SE)

1. A hybridoma cell line that produces a monoclonal antibody which reacts specifically with one species of *Mycosphaerella and* which is capable of detecting *Mycosphaerella* infections in the early stages of infection before disease symptoms appear.

2. A hybridoma cell line that produces a monoclonal antibody which reacts specifically with more than one species of *Mycosphaerella* and which is capable of detecting *Mycosphaerella* infections in the early stages of infection before disease symptoms appear.

3. The hybridoma cell line of claim 1 in which the antibody reacts *specifically* with one species selected from the group consisting of *M. fijiensis, M. fijiensis* var *difformis, M. graminicola* and *M. musicola.*

4. The hybridoma cell line of claim 2 in which the antibody reacts specifically with more than one species selected from the group consisting of *M. fijiensis, M. fijiensis* var *difformis*, *M. graminicola* and *M. musicola.*

5. The hybridoma cell line of claim 3 in which the species is *M. graminicola.*

6. The hybridoma cell line of claim 5 deposited under accession number ATCC HB10186, or a mutant thereof, that is capable of producing a monoclonal antibody which reacts specifically with *M graminicola.*

7. The hybridoma cell line of claim 4 deposited under accession number ATCC HB10413, or a mutant thereof, that is capable of producing a monoclonal antibody which reacts specifically with more than one species selected from the group consisting of *M. fijiensis, M. fijiensis var difformis,* and *M. musicola.*

8. The hybridoma cell line of claim 4 *deposited under accession number* ATCC HB10414, or a mutant thereof, that is capable of producing a monoclonal antibody which reacts specifically with more than one species selected from the group consisting of *M. fijiensis, M. fijiensis var difformis, M. graminicola* and *M. musicola.*

9. A monoclonal antibody for derivatives thereof that reacts specifically with one species of *Mycosphaerella* and which is capable of detecting *Mycosphaerella* infections in the early stages of infection before disease symptoms appear.

10. A monoclonal antibody for derivatives thereof that reacts specifically with more than one species of *Mycosphaerella* and which is capable of detecting *Mycosphaerella* infections in the early stages of infection before disease symptoms appear.

11. The antibody of claim 9 which reacts *specifically* with one species selected from the group consisting of *M. fijiensis, M. fijiensis* var *difformis, M. graminicola* and *M. musicola.*

12. The antibody of claim 10 which reacts specifically with more than one species selected from the group consisting of *M. fijiensis, M. fijiensis var difformis, M. graminicola* and *M. musicola.*

13. The antibody of claim 11 in which the species is *M. graminicola.*

14. The antibody of claim 13 which is produced by a hybridoma cell line deposited under accession number ATCC HB10186, or a mutant thereof, that is capable of producing a monoclonal antibody which reacts specifically with *M graminicola.*

15. The antibody of claim 12 which is produced by a hybridoma cell line deposited under accession number ATCC HB10413, or a mutant thereof, that is capable of producing a monoclonal antibody which reacts specifically with more than one species selected from the group consisting of *M. fijiensis, M. fijiensis var difformis,* and *M. musicola.*

16. The antibody of claim 12 which is produced by a hybridoma cell line deposited under accession number ATCC HB10414, or a mutant thereof, that is capable of producing a monoclonal antibody which reacts specifically with more than one species selected from the group consisting of *M. fijiensis, M. fijiensis var difformis, M. graminicola* and *M. musicola.*

17. The antibody of claim 12 which is produced in response to an extract of mycelia or condidia of *Mycosphaerella fijiensis.*

18. The antibody of claim 13 which is produced in response to an extract of germinated spores of *Mycosphaerella graminicola.*

19. A method of detecting the presence or absence of *Mycosphaerella* in a sample suspected of containing *Mycosphaerella* antigens which comprises contacting the sample with a monoclonal antibody *according to any one of claims 9 to 18* and observing the presence or absence of an antibody-antigen binding reaction.

20. The method of claim 19 which comprises contacting the sample with two antibodies which react with *Mycosphaerella,* wherein one of the antibodies is immobilized, and the other of the antibodies is labelled with a reporter molecule and wherein at least one of the antibodies is a monoclonal antibody according to any one of claims 9 to 18.

21. The method of claims 19 or 20 in which at least on antibody is an antibody according to any one of claims 9, 11, 13, 14 or 18.

22. The method of claims 19 or 20 in which at least one antibody is an antibody according to any one of claims 10, 12, 15, 16 or 17

23. The method of claim 21 in which *at least one of* the antibodies is an antibody according to claim 11.

24. The method of claim 22 in which at least one of the antibodies is an antibody according to claim 12.

25. The method of claim 23 in which at least one of the antibodies is an antibody according to claim 13].

26. The method of claim 20 in which one antibody is monoclonal and one antibody is polyclonal.

27. The method of claim 25 in which at least one of the antibodies is produced by a hybridoma cell line deposited under accession number ATCC HB10186, or a mutant thereof, that is capable of producing a monoclonal antibody which reacts specifically with *M graminicola.*

28. The method of claim 22 in which at least one of the antibodies is produced by a hybridoma cell line deposited under accession number ATCC HB10413, or a mutant thereof, that is capable of producing a monoclonal antibody which reacts specifically with more than one species selected from the group consisting of *M. fijiensis, M. fijiensis var difformis,* and *M. musicola.*

29. The method of claim 22 in which *at least one of* the antibodies is produced by a hybridoma cell line deposited under accession number ATCC HB10414, or a mutant thereof, that is capable of producing a monoclonal antibody which reacts specifically with more than one species selected from the group consisting of *M. fijiensis, M. fijiensis var difformis, M. graminicola* and *M. musicola.*

30. A composition for the immunological detection of the presence or absence of *Mycosphaerella* in a sample suspected of containing *Mycosphaerella* antigens in form of a diagnostic kit comprising an antibody bound to a solid support and an antibody labelled with a reporter molecule, wherein both antibodies are capable of reacting with *Mycosphaerella,* and at least one of the antibodies is a monoclonal antibody according to any one of claims 9 to 18.

31. The composition of claim 30 in which at least on antibody is an antibody according to any one of claims 9, 11, 13, 14 or 18.

32. The composition of claim 31 in which at least one antibody is an antibody according to any one of claims 10, 12, 15, 16 or 17.

33. The composition of claim 31 in which at least one of the antibodies is an antibody according to claim 13.

34. The composition of claim 33 which contains the antibody produced by the hybridoma cell line deposited under accession number ATCC HB10186, or a mutant thereof, that is capable of producing a monoclonal antibody which reacts specifically with *M graminicola.*

35. The composition of claim 32 which contains the antibody produced by the hybridoma cell line deposited under accession number ATCC HB10413, or a mutant thereof, that is capable of producing a monoclonal antibody which reacts specifically with more than one species selected from the group consisting of *M. fijiensis, M. fijiensis var difformis,* and *M. musicola.*

36. The composition of claim 32 which contains the antibody produced by the hybridoma cell line deposited under accession number ATCC HB10414, or a mutant thereof, that is capable of producing a monoclonal antibody which reacts specifically with more than one species selected from the group consisting of *M. fijiensis, M. fijiensis var difformis, M. graminicola* and *M. musicola.*

37. The composition of any one of claims 36 or 37 in which the reporter molecule is an enzyme.

38. The composition of claim 37 which also comprises a substrate for the enzyme.

39. The composition of claim 31 in which at least one antibody is specific to *M. fijiensis.*

40. The composition of claim 31 in which at least one antibody is specific to *M. fijiensis* var. *difformis.*

41. A method for preparing a hybridoma cell line according to any one of claims 1 to 8, which comprises
(a) immunizing a donor animal with an extract of *Mycosphaerella;*
(b) isolating immunocompetent B cells from the immunized donor animal;
(c) fusing the B cells with cells of an immortal cell line;
(d) sceening fusion products and identifying those which produce antibodies having specificity for *Mycosphaerella;*
(e) cloning the fusion product isolated in step (d) to obtain a pure hybridoma cell line which produces a monoclonal antibody having specificity for *Mycosphaerella* and being capable of detecting *Mycosphaerella* infections in the early stages of infection before disease symptoms appear.

42. A method for preparing a monoclonal antibody according to any one of claims 9 to 18 which comprises culturing a hybridoma cell line capable of producing such antibodies under conditions conductive to antibody production, and isolating the antibodies so produced.

43. A method according to claim 42 wherein the cultivation of the hybridoma cell line is carried out *in vivo* or *in vitro.*

## Claims (Claims for the following Contracting State(s): ES)

1. A method for preparing a hybridoma cell line that produces a monoclonal antibody that reacts specifically with one species of the genus *Mycosphaerella,* which comprises:
(a) immunizing a donor animal with an extract of *Mycosphaerella;*
(b) isolating immunocompetentB cells from the immunized donor animal;
(c) fusing the B cells with cells of an immortal cell line;
(d) sceening fusion products and identifying those which produce antibodies having specificity for *Mycosphaerella;*
(e) cloning the fusion product isolated in step (d) to obtain a pure hybridoma cell line which produces a monoclonal antibody having specificity for one species of the genus *Mycosphaerella* and being capable of detecting Mycosphaerella infections in the early stages of infection before disease symptoms appear.

2. A method for preparing a hybridoma cell line that produces a monoclonal antibody which reacts specifically with more than one species of Mycosphaerella and which is capable of detecting Mycosphaerella infections in the early stages of infection before disease symptoms appear, which comprises
(a) immunizing a donor animal with an extract of Mycosphaerella;
(b) isolating immunocompetent B cells from the immunized donor animal;
(c) fusing the B cells with cells of an immortal cell line;
(d) sceening fusion products and identifying those which produce antibodies having specificity for Mycosphaerella;
(e) cloning the fusion product isolated in step (d) to obtain a pure hybridoma cell line which produces a monoclonal antibody having specificity for more than one species of Mycosphaerella and being capable of detecting Mycosphaerella infections in the early stages of infection before disease symptoms appear.

3. A method according to any one of claims 1 or 2, wherein the extract of *Mycosphaerella* is prepared from mycelia or condidia of *Mycosphaerella fijiensis.*

4. A method according to any one of claims 1 or 2, wherein the extract of *Mycosphaerella* is prepared from ungerminated and/or germinated spores of *Mycosphaerella graminicola.*

5. A method according to any one of claims 1 or 2, wherein for inoculation the extracts are combined with an appropriate adjuvant.

6. A method according to any one of claims 1 or 2, wherein the immunization of the donor animal is carried out by single or repeated administration of the antigen extract.

7. A method according to any one of claims 1 or 2, wherein the immortal cell line used in the fusion reaction is a myeloma cell line.

8. A method according to claim 6, wherein the myeloma cell line is one selected from the group consisting of NS-1, SP2/0-Ag14 and X63-Ag8.653.

9. A method according to any one of claims 1 or 2, wherein the fusion is carried out in a buffer solution containing on of the fusion promoters costomarily used for the fusion of cells.

10. A method according to any one of claims 1 or 2, wherein a HAT selection medium is used for the selection of fused hybrid cells.

11. A method according to any one of claims 1 or 2, wherein the screeing of the fusion product for the fomation of suitable monoclonal antibodies is carried out by means of an immunoassay.

12. A method according to any one of claims 1 or 2, wherein a hybridoma cell line is selected that produces a monoclonal antibody which reacts specifically with one species of *Mycosphaerella* selected from the group consisting of *M. fijiensis M. fijiensis* var *difformis, M. graminicola* and *M. musicola.*

13. A method according to any one of claims 1 or 2, wherein a hybridoma cell line is selected that produces a monoclonal antibody which reacts specifically with more than one species of Mycosphaerella selected from the group consisting of *M. fijiensis M. fijiensis var difformis, M. graminicola* and *M. musicola.*

14. A method according to claim 12, wherein the species of *Mycosphaerella* is *M. graminicola.*

15. A method according to claim 14, wherein a hybridoma cell line is selected that has been deposited under accession number ATCC HB10186.

16. A method according to claim 13, wherein a hybridoma cell line is selected that has been deposited under accession number ATCC HB10413.

17. A method according to claim 13, wherein a hybridoma cell line is selected that has been deposited under accession number ATCC HB10414.

18. A method for preparing a monoclonal antibody that reacts specifically with one species of *Mycosphaerella* and is capable of detecting Mycosphaerella infections in the early stages of infection before disease symptoms appear, which comprises culturing a hybridoma cell line capable of producing such antibodies under conditions conducive to antibody production, and isolating the antibodies so produced.

19. A method for preparing a monoclonal antibody that reacts specifically with more than one one species of *Mycosphaerella* and is capable of detecting Mycosphaerella infections in the early stages of infection before disease symptoms appear, which comprises culturing a hybridoma cell line capable of producing such antibodies under conditions conducive to antibody production, and isolating the antibodies so produced.

20. A method according to any one of claims 18 or 19, wherein the cultivation of the hybridoma cell line is carried out *in vivo* or *in vitro.*

21. A method according to claims 18, wherein a monoclonal antibody is produced that reacts with one species of *Mycosphaerella* selected from the group consisting of *M. fijiensis M. fijiensis* var *difformis, M. graminicola* and *M. musicola.*

22. A method according to any one of claims 19, which reacts specifically with more than one species of Mycosphaerella selected from the group consisting of *M. fijiensis M. fijiensis var difformis, M. graminicola* and *M. musicola.*

23. A method according to claim 21, wherein the species of *Mcosphaerella* is *M. graminicola.*

24. A method according to claim 23, wherein a hydridoma cell line is used that has been deposited under accession number ATCC HB10186, or a mutant thereof, that is capable of producing a monoclonal antibody which reacts specifically with *M graminicola.*

25. A method according to claim 22, wherein a hybridoma cell line is used that has been deposited under accession number ATCC HB10413, or a mutant thereof, that is capable of producing a monoclonal antibody which reacts specifically with more than one species selected from the group consisting of *M. fijiensis, M. fijiensis var difformis, and M. musicola.*

26. A method according to claim 22, wherein a hybridoma cell line is used that has been deposited under accession number ATCC HB10414, or a mutant thereof, that is capable of producing a monoclonal antibody which reacts specifically with more than one species selected from the group consisting of *M. fijiensis, M. fijiensis var difformis, M. graminicola and M. musicola.*

27. A method according to claim 22, wherein the monoclonal antibody is produced in response to an extract of mycelia or condidia of *Mycosphaerella fijiensis.*

28. A method according to claim 23, wherein the monoclonal antibody is produced in response to an extract of ungerminated and/or germinated spores of *Mycosphaerella graminicola.*

29. A method of detecting the presence or absence of *Mycosphaerella* in a sample suspected of containing *Mycosphaerella* antigens which comprises contacting the sample with a monoclonal antibody as produced by a process according to any one of claims 18 to 28 or derivatives thereof, and observing the presence or absence of an antibody-antigen binding reaction.

30. The method of claim 29 which comprises contacting the sample with two antibodies which react with *Mycosphaerella,* wherein one of the antibodies is immobilized, and the other of the antibodies is labelled with a reporter molecule and wherein at least one of the antibodies is a monoclonal antibody as produced according to any one of claims 18 to 28.

31. The method of claims 29 or 30 in which at least on antibody is an antibody produced by a process according to any one of claims 18, 20, 21, 23, 24, or 28.

32. The method of claims 29 or 30 in which at least one antibody is an antibody according to any one of claims 19, 20, 22, 25, 26, or 27

33. The method of claim 31 in which at least one of the antibodies is an antibody according to claim 21

34. The method of claim 32 in which at least one of the antibodies is an antibody according to claim 22.

35. The method of claim 33 in which at least one of the antibodies is an antibody according to claim 23.

36. The method of claim 30 in which one antibody is monoclonal and one antibody is polyclonal.

37. The method of claim 35 in which at least one of the antibodies is produced by a hybridoma cell line deposited under accession number ATCC HB10186, or a mutant thereof, that is capable of producing a monoclonal antibody which reacts specifically with *M graminicola.*

38. The method of claim 34 in which at least one of the antibodies is produced by a hybridoma cell line deposited under accession number ATCC HB10413, or a mutant thereof, that is capable of producing a monoclonal antibody which reacts specifically with more than one species selected from the group consisting of *M. fijiensis, M. fijiensis var difformis,* and *M. musicola.*

39. The method of claim 34 in which at least one *of* the antibodies is produced by a hybridoma cell line deposited under accession number ATCC HB10414, or a mutant thereof, that is capable of producing a monoclonal antibody which reacts specifically with more than one species selected from the group consisting of M. fijiensis, M. fijiensis var difformis, M. graminicola and M. musicola.

40. A composition for the immunological detection of the presence or absence of *Mycosphaerella* in a sample suspected of containing *Mycosphaerella* antigens in form of a diagnostic kit comprising an antibody bound to a solid support and an antibody labelled with a reporter molecule, wherein both antibodies are capable of reacting with *Mycosphaerella,* and at least one of the antibodies is a monoclonal antibody prepared according to any one of claims 18 to 28.

41. The composition of claim 40 in which at least on antibody is an antibody prepared according to any one of claims 18, 20, 21, 23, 24 or 28.

42. The composition of claim 41 in which at least one antibody is an antibody prepared according to any one of claims 19, 20, 22, 25, 26, or 27.

43. The composition of claim 41 in which at least one of the antibodies is an antibody according to claim 23.

44. The composition of claim 43 which contains the antibody produced by the hybridoma cell line deposited under accession number ATCC HB10186, or a mutant thereof, that is capable of producing a monoclonal antibody which reacts specifically with *M graminicola.*

45. The composition of claim 42 which contains the antibody produced by the hybridoma cell line deposited under accession number ATCC HB10413, or a mutant thereof, that is capable of producing a monoclonal antibody which reacts specifically with more than one species selected from the group consisting of *M. fijiensis, M. fijiensis var difformis,* and *M. musicola.*

46. The composition of claim 42 which contains the antibody produced by the hybridoma cell line deposited under accession number ATCC HB10414, or a mutant thereof, that is capable of producing a monoclonal antibody which reacts specifically with more than one species selected from the group consisting of *M. fijiensis, M. fijiensis var difformis, M. graminicola* and *M. musicola.*

47. The composition of any one of claims 46 or 47 in which the reporter molecule is an enzyme.

48. The composition of claim 47 which also comprises a substrate for the enzyme.

49. The composition of claim 41 in which at least one antibody is specific to *M. fijiensis.*

50. The composition of claim 41 in which at least one antibody is specific to *M. fijiensis* var*. difformis.*

## Patentansprüche (Patentansprüche für folgende(n) Vertragsstaat(en): AT, BE, CH, DE, DK, FR, GB, GR, IT, LI, LU, NL, SE)

1. Hybridomazellinie, die einen monoclonalen Antikörper, der spezifisch mit einer *Mycosphaerella-*Art reagiert und der *Mycosphaerella*-Infektionen in den frühen Infektionsstadien vor Auftreten von Krankheitssymptomen nachweisen kann, produziert.

2. Hybridomazellinie, die einen monoclonalen Antikörper, der spezifisch mit mehr als einer *Mycosphaerella-*Art reagiert und der *Mycosphaerella*-Infektionen in den frühen Infektionsstadien vor Auftreten von Krankheitssymptomen nachweisen kann, produziert.

3. Hybridomazellinie nach Anspruch 1, wobei der Antikörper spezifisch mit einer Art, ausgewählt aus der Gruppe bestehend aus *M. fijiensis, M. fijiensis* var. *difformis, M.* graminicola und *M. musicola,* reagiert.

4. Hybridomazellinie nach Anspruch 2, wobei der Antikörper spezifisch mit mehr als einer Art, ausgewählt aus der Gruppe, bestehend aus *M. fijiensis, M. fijiensis* var. *difformis, M. graminicola* und *M. musicola,* reagiert.

5. Hybridomazellinie nach Anspruch 3, wobei die Art *M. graminicola* ist.

6. Hybridomazellinie nach Anspruch 5, hinterlegt unter der Hinterlegungsnummer ATCC HB10186, oder eine Mutante davon, die einen monoclonalen Antikörper, der spezifisch mit *M. graminicola* reagiert, produzieren kann.

7. Hybridomazellinie nach Anspruch 4, hinterlegt unter der Hinterlegungsnummer ATCC HB10413, oder eine Mutante davon, die einen monoclonalen Antikörper, der spezifisch mit mehr als einer Art, ausgewählt aus der Gruppe bestehend aus *M. fijiensis, M. fijiensis* var. *difformis* und *M. musicola,* reagiert, produzieren kann.

8. Hybridomazellinie nach Anspruch 4, hinterlegt unter der Hinterlegungsnummer ATCC HB10414, oder eine Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann.

9. Monoclonaler Antikörper oder Derivate davon, der spezifisch mit einer *Mycosphaerella-*Art reagiert und der *Mycosphaerella*-Infektionen in den frühen Infektionsstadien vor Auftreten von Krankheitssymptomen nachweisen kann.

10. Monoclonaler Antikörper oder Derivate davon, der spezifisch mit einer *Mycosphaerella-*Art reagiert und der *Mycosphaerella*-Infektionen in den frühen Infektionsstadien vor Auftreten von Krankheitssymptomen nachweisen kann.

11. Antikörper nach Anspruch 9, der spezifisch mit einer Art, ausgewählt aus der Gruppe bestehend aus *M. fijiensis, M. fijiensis* var. *difformis, M. graminicola* und *M. musicola,* reagiert.

12. Antikörper nach Anspruch 10, der spezifisch mit mehr als einer Art, ausgewählt aus der Gruppe bestehend aus *M. fijiensis, M. fijiensis* var. *difformis, M. graminicola* und *M. musicola,* reagiert.

13. Antikörper nach Anspruch 11, wobei die Art *M. graminicola* ist.

14. Antikörper nach Anspruch 13, der von einer Hybridomazellinie, die unter der Hinterlegungsnummer ATCC HB10186 hinterlegt ist, oder einer Mutante davon, die einen monoclonalen Antikörper, der spezifisch mit *M. graminicola* reagiert, produzieren kann, produziert wird.

15. Antikörper nach Anspruch 12, der von einer Hybridomazellinie, die unter der Hinterlegungsnummer ATCC HB10413 hinterlegt ist, oder einer Mutante davon, die einen monoclonalen Antikörper, der spezifisch mit mehr als einer Art, ausgewählt aus der Gruppe bestehend aus *M. fijiensis, M. fijiensis* var. *difformis und M. musicola,* reagiert, produzieren kann, produziert wird.

16. Antikörper nach Anspruch 12, der von einer Hybridomazellinie, die unter der Hinterlegungsnummer ATCC HB10414 hinterlegt ist, oder einer Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann, produziert wird.

17. Antikörper nach Anspruch 12, der als Antwort auf einen Myzel- oder Konidienextrakt von *Mycosphaerella fijiensis* produziert wird.

18. Antikörper nach Anspruch 13, der als Antwort auf einen Extrakt gekeimter Sporen von *Mycosphaerella graminicola* produziert wird.

19. Verfahren zum Nachweis der Anwesenheit oder Abwesenheit von *Mycosphaerella* in einer Probe, die vermutlich *Mycosphaerella*-Antigene enthält, wobei man die Probe mit einem monoclonalen Antikörper nach einem der Ansprüche 9 bis 18 in Kontakt bringt und die Anwesenheit oder Abwesenheit einer Antikörper-Antigen-Bindungsreaktion beobachtet.

20. Verfahren nach Anspruch 19, wobei man die Probe mit zwei Antikörpern, die mit *Mycosphaerella* reagieren, in Kontakt bringt, wobei einer der Antikörper immobilisiert ist und der andere Antikörper mit einem Reportermolekül markiert ist und wobei mindestens einer der Antikörper ein monoclonaler Antikörper nach einem der Ansprüche 9 bis 18 ist.

21. Verfahren nach den Ansprüchen 19 oder 20, wobei mindestens ein Antikörper ein Antikörper nach einem der Ansprüche 9, 11, 13, 14 oder 18 ist.

22. Verfahren nach den Ansprüchen 19 oder 20, wobei mindestens ein Antikörper ein Antikörper nach einem der Ansprüche 10, 12, 15, 16 oder 17 ist.

23. Verfahren nach Anspruch 21, wobei mindestens einer der Antikörper ein Antikörper nach Anspruch 11 ist.

24. Verfahren nach Anspruch 22, wobei mindestens einer der Antikörper ein Antikörper nach Anspruch 12 ist.

25. Verfahren nach Anspruch 23, wobei mindestens einer der Antikörper ein Antikörper nach Anspruch 13 ist.

26. Verfahren nach Anspruch 20, wobei ein Antikörper monoclonal und ein Antikörper polyclonal ist.

27. Verfahren nach Anspruch 25, wobei mindestens einer der Antikörper von einer Hybridomazellinie, die unter der Hinterlegungsnummer ATCC HB10186 hinterlegt wurde, oder einer Mutante davon, die einen monoclonalen Antikörper, der spezifisch mit *M. graminicola* reagiert, produzieren kann, produziert wird.

28. Verfahren nach Anspruch 22, wobei mindestens einer der Antikörper von einer Hybridomazellinie, die unter der Hinterlegungsnummer ATCC HB10413 hinterlegt wurde, oder einer Mutante davon, die einen monoclonalen Antikörper, der spezifisch mit mehr als einer Art, ausgewählt aus der Gruppe bestehend aus *M. fijiensis, M. fijiensis* var. *difformis* und *M. musicola,* reagiert, produzieren kann, produziert wird.

29. Verfahren nach Anspruch 22, wobei mindestens einer der Antikörper von einer Hybridomazellinie, die unter der Hinterlegungsnummer ATCC HB10414 hinterlegt wurde, oder einer Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann, produziert wird.

30. Zusammensetzung zum immunologischen Nachweis der Anwesenheit oder Abwesenheit von *Mycosphaerella* in einer Probe, die vermutlich *Mycosphaerella*-Antigene enthält, in Form eines diagnostischen Kits, umfassend einen an einen festen Träger gebundenen Antikörper und einen mit einem Reportermolekül markierten Antikörper, wobei beide Antikörper mit *Mycosphaerella* reagieren können und mindestens einer der Antikörper ein monoclonaler Antikörper nach einem der Ansprüche 9 bis 18 ist.

31. Zusammensetzung nach Anspruch 30, wobei mindestens ein Antikörper ein Antikörper nach einem der Ansprüche 9, 11, 13, 14 oder 18 ist.

32. Zusammensetzung nach Anspruch 31, wobei mindestens ein Antikörper ein Antikörper nach einem der Ansprüche 10, 12, 15, 16 oder 17 ist.

33. Zusammensetzung nach Anspruch 31, wobei mindestens einer der Antikörper ein Antikörper nach Anspruch 13 ist.

34. Zusammensetzung nach Anspruch 33, die den Antikörper enthält, der von der Hybridomazellinie, die unter der Hinterlegungsnummer ATCC HB10186 hinterlegt wurde, oder einer Mutante davon, die einen monoclonalen Antikörper, der spezifisch mit *M. graminicola* reagiert, produzieren kann, produziert wird.

35. Zusammensetzung nach Anspruch 32, die den Antikörper enthält, der von der Hybridomazellinie, die unter der Hinterlegungsnummer ATCC HB10413 hinterlegt wurde oder einer Mutante davon, die einen monoclonalen Antikörper, der spezifisch mit mehr als einer Art, ausgewählt aus der Gruppe bestehend aus *M. fijiensis, M. fijiensis* var. *difformis* und *M. musicola,* reagiert, produzieren kann, produziert wird.

36. Zusammensetzung nach Anspruch 32, die den Antikörper enthält, der von der Hybridomazellinie, die unter der Hinterlegungsnummer ATCC HB10414 hinterlegt wurde, oder einer Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann, produziert wird.

37. Zusammensetzung nach einem der Ansprüche 36 oder 37, wobei das Reportermolekül ein Enzym ist.

38. Zusammensetzung nach Anspruch 37, die auch ein Substrat für das Enzym umfaßt.

39. Zusammensetzung nach Anspruch 31, wobei mindestens ein Antikörper für *M. fijiensis* spezifisch ist.

40. Zusammensetzung nach Anspruch 31, wobei mindestens ein Antikörper für *M. fijiensis* var. *difformis* spezifisch ist.

41. Verfahren zur Herstellung einer Hybridomazellinie nach einem der Ansprüche 1 bis 8, wobei man
(a) ein Spendertier mit einem *Mycosphaerella-*Extrakt immunisiert;
(b) immunkompetente B-Zellen aus dem immunisierten Spendertier isoliert;
(c) die B-Zellen mit Zellen einer unsterblichen Zellinie fusioniert;
(d) Fusionsprodukte absucht und diejenigen identifiziert, die Antikörper mit *Mycosphaerella*-Spezifität produzieren;
(e) das in Stufe (d) isolierte Fusionsprodukt cloniert, um eine reine Hybridomazellinie zu erhalten, die einen monoclonalen Antikörper mit *Mycosphaerella-*Spezifität*,* der *Mycosphaerella*-Infektionen in den frühen Infektionsstadien vor Auftreten von Krankheitssymptomen nachweisen kann, produziert.

42. Verfahren zur Herstellung eines monoclonalen Antikörpers nach einem der Ansprüche 9 bis 18, wobei man eine Hybridomazellinie, die solche Antikörper produzieren kann, unter Bedingungen, die zur Antikörperproduktion führen, produziert und die so produzierten Antikörper isoliert.

43. Verfahren nach Anspruch 42, wobei die Züchtung der Hybridomazellinie *in vivo* oder *in vitro* durchgeführt wird.

## Patentansprüche (Patentansprüche für folgende(n) Vertragsstaat(en): ES)

1. Verfahren zur Herstellung einer Hybridomazellinie, die einen monoclonalen Antikörper produziert, der spezifisch mit einer Art der Gattung *Mycosphaerella* reagiert, wobei man
(a) ein Spendertier mit einem *Mycosphaerella-*Extrakt immunisiert;
(b) immunkompetente B-Zellen aus dem immunisierten Spendertier isoliert;
(c) die B-Zellen mit Zellen einer unsterblichen Zellinie fusioniert;
(d) Fusionsprodukte absucht und diejenigen identifiziert, die Antikörper mit *Mycosphaerella*-Spezifität produzieren;
(e) das in Stufe (d) isolierte Fusionsprodukt cloniert, um eine reine Hybridomazellinie zu erhalten, die einen monoclonalen Antikörper mit Spezifität für eine Art der Gattung *Mycosphaerella,* der *Mycosphaerella*-Infektionen in den frühen Infektionsstadien vor Auftreten von Krankheitssymptomen nachweisen kann, produziert.

2. Verfahren zur Herstellung einer Hybridomazellinie, die einen monoclonalen Antikörper produziert, der spezifisch mit mehr als einer *Mycosphaerella-*Art reagiert und der *Mycosphaerella*-Infektionen in den frühen Infektionsstadien vor Auftreten von Krankheitssymptomen nachweisen kann, wobei man
(a) ein Spendertier mit einem *Mycosphaerella*-Extrakt immunisiert;
(b) immunkompetente B-Zellen aus dem immunisierten Spendertier isoliert;
(c) die B-Zellen mit Zellen einer unsterblichen Zellinie fusioniert;
(d) Fusionsprodukte absucht und diejenigen identifiziert, die Antikörper mit *Mycosphaerella-*Spezifität produzieren;
(e) das in Stufe (d) isolierte Fusionsprodukt cloniert, um eine reine Hybridomazellinie zu erhalten, die einen monoclonalen Antikörper mit Spezifität für mehr als eine *Mycosphaerella-*Art*,* der *Mycosphaerella*-Infektionen in den frühen Infektionsstadien vor Auftreten von Krankheitssymptomen nachweisen kann, produziert.

3. Verfahren nach einem der Ansprüche 1 oder 2, wobei man den *Mycosphaerella-*Extrakt aus Myzelien oder Konidien von *Mycosphaerella fijiensis* herstellt.

4. Verfahren nach einem der Ansprüche 1 oder 2, wobei man den *Mycosphaerella-*Extrakt aus ungekeimten und/oder gekeimten Sporen von *Mycosphaerella graminicola* herstellt.

5. Verfahren nach einem der Ansprüche 1 oder 2, wobei man zur Inokulierung die Extrakte mit einem geeigneten Adjuvans vereinigt.

6. Verfahren nach einem der Ansprüche 1 oder 2, wobei man die Immunisierung des Spendertieres durch einzelne oder wiederholte Verabreichung(en) des Antigenextrakts durchführt.

7. Verfahren nach einem der Ansprüche 1 oder 2, wobei die bei der Fusionsreaktion verwendete unsterbliche Zellinie eine Myelomazellinie ist.

8. Verfahren nach Anspruch 6, wobei die Myelomazellinie eine Zellinie, ausgewählt aus der Gruppe bestehend aus NS-1, SP2/0-Ag14 und X63-Ag8.653, ist.

9. Verfahren nach einem der Ansprüche 1 oder 2, wobei man die Fusion in einer Pufferlösung, die einen der üblicherweise für Zellfusionen verwendeten Fusionspromotoren enthält, durchführt.

10. Verfahren nach einem der Ansprüche 1 oder 2, wobei man ein HAT-Selektionsmedium zur Selektion von fusionierten Hybridzellen verwendet.

11. Verfahren nach einem der Ansprüche 1 oder 2, wobei man das Screening des Fusionsprodukts auf die Bildung geeigneter monoclonaler Antikörper mit einem Immunoassay durchführt.

12. Verfahren nach einem der Ansprüche 1 oder 2, wobei man eine Hybridomazellinie auswählt, die einen monoclonalen Antikörper produziert, der spezifisch mit einer *Mycosphaerella-*Art, ausgewählt aus der Gruppe bestehend aus *M. fijiensis, M. fijiensis* var. *difformis, M. graminicola* und *M. musicola,* reagiert.

13. Verfahren nach einem der Ansprüche 1 oder 2, wobei man eine Hybridomazellinie auswählt, die einen monoclonalen Antikörper, der spezifisch mit mehr als einer *Mycosphaerella-*Art*,* ausgewählt aus der Gruppe bestehend aus *M. fijiensis, M. fijiensis* var. *difformis, M. graminicola* und *M. musicola,* reagiert, produziert.

14. Verfahren nach Anspruch 12, wobei die *Mycosphaerella-*Art *M. graminicola* ist*.*

15. Verfahren nach Anspruch 14, wobei man eine Hybridomazellinie, die unter Hinterlegungsnummer ATCC HB10186 hinterlegt wurde, auswählt.

16. Verfahren nach Anspruch 13, wobei man eine Hybridomazellinie, die unter Hinterlegungsnummer ATCC HB10413 hinterlegt wurde, auswählt.

17. Verfahren nach Anspruch 13, wobei man eine Hybridomazellinie, die unter Hinterlegungsnummer ATCC HB10414 hinterlegt wurde, auswählt.

18. Verfahren zur Herstellung eines monoclonalen Antikörpers, der spezifisch mit einer *Mycosphaerella-*Art reagiert und der *Mycosphaerella*-Infektionen in den frühen Infektionsstadien vor Auftreten von Krankheitssymptomen nachweisen kann, wobei man eine Hybridomazellinie, die solche Antikörper produzieren kann, unter Bedingungen, die zur Antikörperproduktion führen, züchtet und die so produzierten Antikörper isoliert.

19. Verfahren zur Herstellung eines monoclonalen Antikörpers, der spezifisch mit mehr als einer *Mycosphaerella*-Art reagiert und der *Mycosphaerella*-Infektionen in den frühen Infektionsstadien vor Auftreten von Krankheitssymptomen nachweisen kann, wobei man eine Hybridomazellinie, die solche Antikörper produzieren kann, unter Bedingungen, die zur Antikörperproduktion führen, züchtet und die so produzierten Antikörper isoliert.

20. Verfahren nach einem der Ansprüche 18 oder 19, wobei man die Züchtung der Hybridomazellinie *in vivo* oder *in vitro* durchführt.

21. Verfahren nach Anspruch 18, wobei ein monoclonaler Antikörper produziert wird, der spezifisch mit einer *Mycosphaerella-*Art*,* ausgewählt aus der Gruppe bestehend aus *M. fijiensis, M. fijiensis* var. *difformis, M. graminicola* und *M. musicola,* reagiert.

22. Verfahren nach Anspruch 19, wobei ein monoclonaler Antikörper produziert wird, der spezifisch mit mehr als einer *Mycosphaerella-*Art*,* ausgewählt aus der Gruppe bestehend aus *M. fijiensis, M. fijiensis* var. *difformis, M. graminicola* und *M. musicola,* reagiert.

23. Verfahren nach Anspruch 21, wobei die *Mycosphaerella-*Art *M. graminicola* ist.

24. Verfahren nach Anspruch 23, wobei man eine Hybridomazellinie, die unter der Hinterlegungsnummer ATCC HB10186 hinterlegt wurde, oder eine Mutante davon, die einen monoclonalen Antikörper, der spezifisch mit *M. graminicola* reagiert, produzieren kann, verwendet.

25. Verfahren nach Anspruch 22, wobei man eine Hybridomazellinie, die unter Hinterlegungsnummer ATCC HB10413 hinterlegt wurde, oder eine Mutante davon, die einen monoclonalen Antikörper, der spezifisch mit mehr als einer Art, ausgewählt aus der Gruppe bestehend aus *M. fijiensis, M. fijiensis* var. *difformis* und *M. musicola,* reagiert, produzieren kann, verwendet.

26. Verfahren nach Anspruch 22, wobei man eine Hybridomazellinie, die unter der Hinterlegungsnummer ATCC HB10414 hinterlegt wurde, oder eine Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann, verwendet.

27. Verfahren nach Anspruch 22, wobei der monoclonale Antikörper als Antwort auf einen Myzel- oder Konidienextrakt von *Mycosphaerella fijiensis* produziert wird.

28. Verfahren nach Anspruch 23, wobei der monoclonale Antikörper als Antwort auf einen Extrakt ungekeimter und/oder gekeimter Sporen von *Mycosphaerella graminicola* produziert wird.

29. Verfahren zum Nachweis der Anwesenheit oder Abwesenheit von *Mycosphaerella* in einer Probe, die vermutlich *Mycosphaerella-*Antigene enthält, wobei man die Probe mit einem nach einem Verfahren der Ansprüche 18 bis 28 produzierten Antikörper oder Derivaten davon in Kontakt bringt und die Anwesenheit oder Abwesenheit einer Antikörper-Antigen-Bindungsreaktion beobachtet.

30. Verfahren nach Anspruch 29, wobei man die Probe mit zwei Antikörpern, die mit *Mycosphaerella* reagieren, in Kontakt bringt, wobei einer der Antikörper immobilisiert ist und der andere Antikörper mit einem Reportermolekül markiert ist, wobei mindestens einer der Antikörper ein monoclonaler Antikörper, produziert nach einem der Ansprüche 18 bis 28, ist.

31. Verfahren nach den Ansprüchen 29 oder 30, wobei mindestens ein Antikörper ein nach einem Verfahren nach einem der Ansprüche 18, 20, 21, 23, 24 oder 28 produzierter Antikörper ist.

32. Verfahren nach den Ansprüchen 29 oder 30, wobei mindestens ein Antikörper ein nach einem der Ansprüche 19, 20, 22, 25, 26 oder 27 produzierter Antikörper ist.

33. Verfahren nach Anspruch 31, wobei mindestens einer der Antikörper ein Antikörper nach Anspruch 21 ist.

34. Verfahren nach Anspruch 32, wobei mindestens einer der Antikörper ein Antikörper nach Anspruch 22 ist.

35. Verfahren nach Anspruch 33, wobei mindestens einer der Antikörper ein Antikörper nach Anspruch 23 ist.

36. Verfahren nach Anspruch 30, wobei ein Antikörper monoclonal und ein Antikörper polyclonal ist.

37. Verfahren nach Anspruch 35, wobei mindestens einer der Antikörper von einer Hybridomazellinie, die unter der Hinterlegungsnummer ATCC HB10186 hinterlegt wurde, oder eine Mutante davon, die einen monoclonalen Antikörper, der spezifisch mit *M. graminicola* reagiert, produzieren kann, produziert wird.

38. Verfahren nach Anspruch 34, wobei mindestens einer der Antikörper von einer Hybridomazellinie, die unter der Hinterlegungsnummer ATCC HB10413 hinterlegt wurde, oder eine Mutante davon, die einen monoclonalen Antikörper, der spezifisch mit mehr als einer Art, ausgewählt aus der Gruppe bestehend aus *M. fijiensis, M. fijiensis* var. *difformis* und *M. musicola,* reagiert, produzieren kann, produziert wird.

39. Verfahren nach Anspruch 34, wobei mindestens einer der Antikörper von einer Hybridomazellinie, die unter der Hinterlegungsnummer ATCC HB10414 hinterlegt wurde, oder einer Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann, produziert wird.

40. Zusammensetzung zum immunologischen Nachweis der Anwesenheit oder Abwesenheit von *Mycosphaerella* in einer Probe, die vermutlich *Mycosphaerella*-Antigene enthält, in Form eines diagnostischen Kits, umfassend einen an einen festen Träger gebundenen Antikörper und einen mit einem Reportermolekül markierten Antikörper, wobei beide Antikörper mit *Mycosphaerella* reagieren können und mindestens einer der Antikörper ein monoclonaler Antikörper nach einem der Ansprüche 18 bis 28 ist.

41. Zusammensetzung nach Anspruch 40, wobei mindestens ein Antikörper ein Antikörper, hergestellt nach einem der Ansprüche 18, 20, 21, 23, 24 oder 28, ist.

42. Zusammensetzung nach Anspruch 41, wobei mindestens ein Antikörper ein Antikörper, hergestellt nach einem der Ansprüche 19, 20, 22, 25, 26 oder 27, ist.

43. Zusammensetzung nach Anspruch 41, wobei mindestens einer der Antikörper ein Antikörper nach Anspruch 23 ist.

44. Zusammensetzung nach Anspruch 43, die einen Antikörper, der von der Hybridomazellinie, die unter Hinterlegungsnummer ATCC HB10186 hinterlegt wurde, oder einer Mutante davon, die einen monoclonalen Antikörper, der spezifisch mit *M. graminicola* reagiert, produzieren kann, produziert wird, enthält.

45. Zusammensetzung nach Anspruch 42, die den Antikörper enthält, der von der Hybridomazellinie, die unter der Hinterlegungsnummer ATCC HB10413 hinterlegt wurde, oder einer Mutante davon, die einen monoclonalen Antikörper produzieren kann, der spezifisch mit mehr als einer Art, ausgewählt aus der Gruppe bestehend aus *M. fijiensis, M. fijiensis* var. *difformis* und *M. musicola,* reagiert, produzieren kann, produziert wird.

46. Zusammensetzung nach Anspruch 42, die den Antikörper, der von der Hybridomazellinie, die unter der Hinterlegungsnummer ATCC HB10414 hinterlegt wurde, oder einer Mutante davon, die einen monoclonalen Antikörper, der spezifisch mit mehr als einer Art, ausgewählt aus der Gruppe bestehend aus *M. fijiensis, M. fijiensis* var. *difformis, M. graminicola* und *M. musicola,* reagiert, produzieren kann, produziert wird.

47. Zusammensetzung nach einem der Ansprüche 46 oder 47, wobei das Reportermolekül ein Enzym ist.

48. Zusammensetzung nach Anspruch 47, die auch ein Substrat für das Enzym enthält.

49. Zusammensetzung nach Anspruch 41, wobei mindestens ein Antikörper für *M. fijiensis* spezifisch ist.

50. Zusammensetzung nach Anspruch 41, wobei mindestens ein Antikörper für *M. fijiensis* var. *difformis* spezifisch ist.

## Revendications (Revendications pour l'(les) Etat(s) contractant(s) suivant(s): AT, BE, CH, DE, DK, FR, GB, GR, IT, LI, LU, NL, SE)

1. Lignée cellulaire d'hybridome qui produit un anticorps monoclonal réagissant spécifiquement avec une espèce de Mycosphaerella et qui est capable de détecter des infections à Mycosphaerella aux stades précoces de l'infection avant l'apparition des symptômes de la maladie.

2. Lignée cellulaire d'hybridome qui produit un anticorps monoclonal réagissant spécifiquement avec plus d'une espèce de Mycosphaerella et qui est capable de détecter les infections à Mycosphaerella aux stades précoces de l'infection avant l'apparition des symptômes de la maladie.

3. Lignée cellulaire d'hybridome de la revendication 1, dans laquelle l'anticorps réagit spécifiquement avec une espèce choisie dans le groupe constitué par M. fijiensis, M. fijiensis var. difformis, M. graminicola et M. musicola.

4. Lignée cellulaire d'hybridome de la revendication 2, dans laquelle l'anticorps réagit spécifiquement avec plus d'une espèce choisie dans le groupe constitué par M. fijiensis, M. fijiensis var. difformis, M. graminicola et M. musicola.

5. Lignée cellulaire d'hydridome de la revendication 3, dans laquelle l'espèce est M. graminicola.

6. Lignée cellulaire d'hybridome de la revendication 5, déposée sous le numéro d'accès ATCC HB10186, ou un de ses mutants, qui est capable de produire un anticorps monoclonal réagissant spécifiquement avec M. graminicola.

7. Lignée cellulaire d'hybridome de la revendication 4, déposée sous le numéro d'accès ATCC HB1 041 3, ou un de ses mutants, qui est capable de produire un anticorps monoclonal réagissant spécifiquement avec plus d'une espèce choisie dans le groupe constitué par M. fijiensis, M. fijensis var. difformis et M. musicola.

8. Lignée cellulaire d'hybridome de la revendication 4, déposée sous le numéro d'accès ATCC HB10414, ou un de ses mutants, qui est capable de produire un anticorps monoclonal réagissant spécifiquement avec plus d'une espèce choisie dans le groupe constitué par M. fijiensis, M. fijiensis var. difformis, M. graminicola et M. musicola.

9. Anticorps monoclonal ou ses dérivés, réagissant spécifiquement avec une espèce de Mycosphaerella et qui est capable de détecter les infections à Mycosphaerella aux stades précoces de l'infection avant l'apparition des symptômes de la maladie.

10. Anticorps monoclonal ou ses dérivés, réagissant spécifiquement avec plus d'une espèce de Mycosphaerella et qui est capable de détecter les infections à Mycosphaerella aux stades précodes de l'infection avant l'apparition des symptômes de la maladie.

11. Anticorps de la revendication 9 qui réagit spécifiquement avec une espèce choisie dans le groupe constitué par M. fijiensis, M. fijiensis var. difformis, M. graminicola et M. musicola.

12. Anticorps de la revendication 10 qui réagit spécifiquement avec plus d'une espèce choisie dans le groupe constitué par M. fijiensis, M. fijiensis var. difformis, M. graminicola et M. musicola.

13. Anticorps de la revendication 11 où l'espèce est M. graminicola.

14. Anticorps de la revendication 13 qui est produit par une lignée cellulaire d'hybridome déposée sous le numéro d'accès ATCC HB10186, ou un de ces mutants, qui est capable de produire un anticorps monoclonal réagissant spécifiquement avec M. graminicola.

15. Anticorps de la revendication 12 qui est produit par une lignée cellulaire d'hybridome déposée sous le numéro d'accès ATCC HB10413, ou un de ses mutants, qui est capable de produire un anticorps monoclonal réagissant spécifiquement avec plus d'une espèce choisie dans le groupe constitué de M. fijiensis, M. fijiensis var. difformis et M. musicola.

16. Anticorps de la revendication 12 qui est produit par une lignée cellulaire d'hybridome déposée sous le numéro d'accès ATCC HB10414, ou un de ses mutants, qui est capable de produire un anticorps monoclonal réagissant spécifiquement avec plus d'une espèce choisie dans le groupe constitué par M. fijiensis, M. fijiensis var. difformis, M. graminicola et M. musicola.

17. Anticorps de la revendication 12, qui est produit en réponse à un extrait de mycélia ou de conidies de Mycosphaerella fijiensis.

18. Anticorps de la revendication 13, qui est produit en réponse à un extrait de spores germées de Mycosphaerella graminicola.

19. Procédé de détection de la présence ou de l'absence de Mycosphaerella dans un échantillon dont on soupçonne qu'il contient les antigènes de Mycosphaerella, dans lequel on met en contact l'échantillon avec un anticorps monoclonal selon l'une quelconque des revendications 9 à 18 et on observe la présence ou l'absence d'une réaction de liaison anticorps-antigène.

20. Procédé de la revendication 19 dans lequel on met en contact l'échantillon avec deux anticorps qui réagissent avec Mycosphaerella, où l'un des anticorps est immobilisé, et l'autre anticorps est marqué avec une molécule rapporteuse et où au moins l'un des anticorps est un anticorps monoclonal selon l'une quelconque des revendications 9 à 18.

21. Procédé des revendications 19 ou 20 dans lequel au moins un anticorps est un anticorps selon l'une quelconque des revendications 9, 11, 13, 14 ou 18.

22. Procédé des revendications 19 ou 20, dans lequel au moins un anticorps est un anticorps selon l'une quelconque des revendications 10, 12, 15, 16 ou 17.

23. Procédé de la revendication 21, dans lequel au moins l'un des anticorps est un anticorps selon la revendication 11.

24. Procédé de la revendication 22, dans lequel au moins l'un des anticorps est un anticorps selon la revendication 12.

25. Procédé de la revendication 23, dans lequel au moins l'un des anticorps est un anticorps selon la revendication 13.

26. Procédé de la revendication 20, dans lequel au moins l'un des anticorps est monoclonal et l'autre anticorps est polyclonal.

27. Procédé de la revendication 25, dans lequel l'un des anticorps est produit par une lignée cellulaire d'hybridome déposée sous le numéro d'accès ATCC HB10186, ou un de ses mutants, qui est capable de produire un anticorps monoclonal réagissant spécifiquement avec M. graminicola.

28. Procédé de la revendication 22, dans lequel au moins l'un des anticorps est produit par une lignée cellulaire d'hybridome déposée sous le numéro d'accès ATCC HB10413, ou un de ces mutants, qui est capable de produire un anticorps monoclonal réagissant spécifiquement avec plus d'une espèce choisie dans le groupe constitué par M. fijiensis, M. fijiensis var. difformis, et M. musicola.

29. Procédé de la revendication 22, dans lequel au moins l'un des anticorps est produit par une lignée cellulaire d'hybridome déposée sous le numéro d'accès ATCC HV10414, ou un de ses mutants, qui est capable de produire un anticorps monoclonal réagissant spécifiquement avec plus d'une espèce choisie dans le groupe constitué par M. fijiensis, M. fijiencis var. difformis, M. graminicola et M. musicola.

30. Composition pour la détection immunologique de la présence ou de l'absence de Mycosphaerella dans un échantillon dont on suspecte qu'il contient des antigènes de Mycosphaerella sous la forme d'une trousse de diagnostic comprenant un anticorps lié à un support solide et un anticorps marqué avec une molécule rapporteuse, où les deux anticorps sont capables de réagir avec Mycosphaerella, et au moins un des anticorps est un anticorps monoclonal selon l'une quelconque des revendications 9 à 18.

31. Composition de la revendication 30 dans laquelle au moins un anticorps est un anticorps selon l'une quelconque des revendications 9, 11, 13, 14 ou 18.

32. Composition de la revendication 31, dans laquelle au moins un anticorps est un anticorps selon l'une quelconque des revendications 10, 12, 15, 16 ou 17.

33. Composition de la revendication 31, dans laquelle au moins l'un des anticorps est un anticorps selon la revendication 13.

34. Composition de la revendication 33, qui contient l'anticorps produit par la lignée cellulaire d'hybridome déposée sous le numéro d'accès ATCC HB10186, ou un de ses mutants qui est capable de produire un anticorps monoclonal réagissant spécifiquement avec M. graminicola.

35. Composition de la revendication 32, qui contient l'anticorps produit par la lignée cellulaire d'hybridome déposée sous le numéro d'accès ATCC HB10413, ou un de ces mutants, qui est capable de produire un anticorps monoclonal réagissant spécifiquement avec plus d'une espèce choisie dans le groupe constitué par M. fijiensis. M. fijiensis var. difformis et M. musicola.

36. Composition de la revendication 32, qui contient l'anticorps produit par la lignée cellulaire d'hybridome déposée sous le numéro d'accès ATCC HB10414, ou un de ses mutants, qui est capable de produire un anticorps monoclonal réagissant spécifiquement avec plus d'une espèce choisie dans le groupe consistué par M. fijiensis. M. fijiensis var. difformis, M. graminicola et M. musicola.

37. Composition de l'une quelconque des revendications 35 ou 36 dans laquelle la molécule rapporteuse est une enzyme.

38. Composition de la revendication 37, qui comprend également un substrat pour l'enzyme.

39. Composition de la revendication 31, dans laquelle au moins un anticorps est spécifique de M. fijiensis.

40. Composition de la revendication 31, dans laquelle au moins un anticorps est spécifique de M. fijiensis var. difformis.

41. Procédé de préparation d'une lignée cellulaire d'hybridome selon l'une quelconque des revendications 1 à 8, dans lequel:
(a) on immunise un animal donneur avec un extrait de Mycosphaerella ;
(b) on isole des cellules B immunocompétentes à partir de l'animal donneur immunisé;
(c) on fusionne des cellules B avec des cellules d'une lignée cellulaire immortelle;
(d) on crible les produits de fusion et on identifie ceux qui produisent des anticorps ayant une spécificité pour Mycosphaerella ;
(e) on clone le produit de fusion isolé dans l'étape (d) pour obtenir une lignée cellulaire d'hybridome pure qui produit un anticorps monoclonal ayant une spécificité pour Mycosphaerella et qui est capable de détecter les infections à Mycosphaerella aux stades précoces de l'infection avant l'apparition des symptômes de la maladie.

42. Procédé de préparation d'un anticorps monoclonal selon l'une quelconque des revendications 9 à 18 dans lequel on cultive une lignée cellulaire d'hybridome capable de produire de tels anticorps sous des conditions conduisant à la production d'anticorps, et on isole les anticorps ainsi produits.

43. Procédé selon la revendication 42, dans lequel la culture de la lignée cellulaire d'hybridome s'effectue in vivo ou in vitro.

## Revendications (Revendications pour l'(les) Etat(s) contractant(s) suivant(s): ES)

1. Procédé de préparation d'une lignée cellulaire d'hybridome qui produit un anticorps monoclonal réagissant spécifiquement avec une espèce du genre Mycosphaerella, dans lequel:
(a) on immunise un animal donneur avec un extrait de Mycosphaerella ;
(b) on isole les cellules B immunocompétentes à partir de l'animal donneur immunisé;
(c) on fusionne les cellules B avec des cellules d'une lignée cellulaire immortelle;
(d) on crible les produits de fusion et on identifie ceux qui produisent des anticorps ayant une spécificité pour Mycosphaerella;
(e) on clone le produit de fusion isolé dans l'étape (d) pour obtenir une lignée cellulaire d'hybridome pure qui produit un anticorps monoclonal ayant une spécificité pour une espèce du genre Mycosphaerella et qui est capable de détecter les infections à Mycosphaerella aux stades précoces de l'infection avant l'apparition des symptômes de la maladie.

2. Procédé pour la préparation d'une lignée cellulaire d'hybridome produisant un anticorps monoclonal qui réagit spécifiquement avec plus d'une espèce de Mycosphaerella et qui est capable de détecter les infections de Mycosphaerella aux stades précoces de l'infection avant l'apparition des symptômes de la maladie, dans lequel:
(a) on immunise un animal donneur avec un extrait de Mycosphaerella ;
(b) on isole les cellules B immunocompétentes à partir de l'animal donneur immunisé;
(c) on fusionne les cellules B avec des cellules d'une lignée cellulaire immortelle;
(d) on crible les produits de fusion et on identifie ceux qui produisent des anticorps ayant une spécificité pour Mycosphaerella;
(e) on clone le produit de fusion isolé dans l'étape (d) pour obtenir une lignée cellulaire d'hybridome pure qui produit un anticorps monoclonal ayant une spécificité pour plus d'une espèce de Mycosphaerella et qui est capable de détecter les infections à Mycosphaerella aux stades précoces de l'infection avant l'apparition des symptômes de la maladie.

3. Procédé selon l'une quelconque des revendications 1 ou 2, dans lequel on prépare l'extrait de Mycosphaerella à partir de mycélia ou de conidies de Mycosphaerella fijiensis.

4. Procédé selon l'une quelconque des revendications 1 ou 2, dans lequel on prépare l'extrait de Mycosphaerella à partir de spores non germées et/ou germées de Mycosphaerella graminicola.

5. Procédé selon l'une quelconque des revendications 1 ou 2, dans lequel pour l'inoculation on combine les extraits avec un adjuvant approprié.

6. Procédé selon l'une quelconque des revendications 1 ou 2, dans lequel on procède à l'immunisation de l'animal donneur par une administration simple ou répétée de l'extrait d'antigène.

7. Procédé selon l'une quelconque des revendications 1 ou 2, dans lequel la lignée cellulaire immortelle utilisée dans la réaction de fusion est une lignée cellulaire de myélome.

8. Procédé selon la revendication 6, dans lequel la lignée cellulaire de myélome est une lignée choisie dans le groupe constitué par NS-1, SPE/0-Ag14 et X63-Ag8.653.

9. Procédé selon l'une quelconque des revendications 1 ou 2, dans lequel on procède à la fusion dans une solution tampon contenant l'un des promoteurs de fusion habituellement utilisé pour la fusion des cellules.

10. Procédé selon l'une quelconque des revendications 1 ou 2, dans lequel on utilise un milieu de sélection HAT pour la sélection des cellules hybrides fusionnées.

11. Procédé selon l'une quelconque des revendications 1 ou 2, dans lequel on procède au criblage du produit de fusion pour la formation d'anticorps monoclonaux appropriés au moyen d'un immunoessai.

12. Procédé selon l'une quelconque des revendications 1 ou 2, dans lequel on sélectionne une lignée cellulaire d'hybridome qui produit un anticorps monoclonal, réagissant spécifiquement avec une espèce de Mycosphaerella choisie dans le groupe constitué par M. fijiensis, M. fijiensis var. difformis, M. graminicola et M. musicola.

13. Procédé selon l'une quelconque des revendications 1 ou 2, dans lequel on sélectionne une lignée cellulaire d'hybridome qui produit un anticorps monoclonal réagissant spécifiquement avec plus d'une espèce de Mycosphaerella choisie dans le groupe constitué de M. fijiensis, M. fijiensis var. difformis, M. graminicola et M. musicola.

14. Procédé selon la revendication 12, dans lequel l'espèce de Mycosphaerella est M. graminicola.

15. Procédé selon la revendication 14, dans lequel on sélectionne une lignée cellulaire d'hybridome qui a été déposée sous le numéro d'accès ATCC HB10168.

16. Procédé selon la revendication 13, dans lequel on sélectionne une lignée cellulaire d'hybridome qui a été déposée sous le numéro d'accès ATCC HB10413.

17. Procédé selon la revendication 13, dans lequel on sélectionne une lignée cellulaire d'hybridome qui a été déposée sous le numéro d'accès ATCC HB10414.

18. Procédé de préparation d'un anticorps monoclonal qui réagit spécifiquement avec une espèce de Mycosphaerella et est capable de détecter les infections à Mycosphaerella aux stades précoces de l'infection avant l'apparition des symptômes de la maladie, dans lequel on cultive une lignée cellulaire d'hybridome capable de produire de tels anticorps dans des conditions conduisant à la production d'anticorps, et on isole les anticorps ainsi produits.

19. Procédé de préparation d'un anticorps monoclonal réagissant spécifiquement avec plus d'une espèce de Mycosphaerella et qui est capable de détecter les infections à Mycosphaerella aux stades précoces de l'infection avant l'apparition des symptômes de la maladie, dans lequel on cultive une lignée cellulaire d'hybridome capable de produire de tels anticorps dans des conditions conduisant à la production d'anticorps, et on isole les anticorps ainsi produits.

20. Procédé selon l'une quelconque des revendications 18 ou 19, dans lequel on procède à la culture de la lignée cellulaire d'hybridome in vivo ou in vitro.

21. Procédé selon la revendication 18, dans lequel on produit un anticorps monoclonal qui réagit avec une espèce de Mycosphaerella choisie dans le groupe constitué par M. fijiensis, M. fijiensis var. difformis, M. graminicola et M. musicola.

22. Procédé selon l'une quelconque des revendications 19-20, où l'anticorps réagit spécifiquement avec plus d'une espèce de Mycosphaerella choisie dans le groupe constitué de M. fijiensis, M. fijiensis var. difformis, M. graminicola et M. musicola.

23. Procédé selon la revendication 21, dans lequel l'espèce de Mycosphaerella est M. graminicola.

24. Procédé selon la revendication 23, dans lequel on utilise une lignée cellulaire d'hybridome qui a été déposée sous le numéro d'accès ATCC HB10186, ou un de ses mutants, qui est capable de produire un anticorps monoclonal réagissant spécifiquement avec M. graminicola.

25. Procédé selon la revendication 22, dans lequel on utilise une lignée cellulaire d'hybridome qui a été déposée sous le numéro d'accès ATCC HB10413, ou un de ces mutants, qui est capable de produire un anticorps monoclonal réagissant spécifiquement avec plus d'une espèce choisie dans le groupe constitué par M. fijiensis. M. fijiensis var. difformis, M. musicola.

26. Procédé selon la revendication 22, dans lequel on utilise une lignée cellulaire d'hybridome déposée sous le numéro d'accès ATCC HV10414, ou un de ses mutants, qui est capable de produire un anticorps monoclonal réagissant spécifiquement avec plus d'une espèce choisie dans le groupe constitué par M. fijiensis, M. fijiensis var. difformis, M. graminicola et M. musicola.

27. Procédé selon la revendication 22, dans lequel l'anticorps monoclonal est produit en réponse à un extrait de mycélia ou de conidies de Mycosphaerella fijiensis.

28. Procédé selon la revendication 23, dans lequel l'anticorps monoclonal est produit en réponse à un extrait de spores non-germées et/ou germées de Mycosphaerella graminicola.

29. Procédé de détection de la présence ou l'absence de Mycosphaerella dans un échantillon dont on soupçonne qui contient des antigènes de Mycosphaerella, dans lequel on met en contact l'échantillon avec un anticorps monoclonal tel que produit par un procédé selon l'une quelconque des revendications 18 à 28, ou un de ses dérivés et on observe la présence ou l'absence d'une réaction de liaison anticorps-antigène.

30. Procédé de la revendication 29, dans lequel on met en contact l'échantillon avec deux anticorps qui réagissent avec Mycosphaerella, où l'un des anticorps est immobilisé, et l'autre anticorps est marqué avec une molécule rapporteuse et où au moins un des anticorps est un anticorps monoclonal, tel que produit selon l'une quelconque des revendications 18 à 28.

31. Procédé des revendications 29 ou 30, dans lequel au moins un anticorps est un anticorps produit par un procédé selon l'une quelconque des revendications 18, 20, 21, 23, 24 ou 28.

32. Procédé des revendications 29 ou 30, dans laquelle au moins un anticorps est un anticorps selon l'une quelconque des revendications 19, 20, 22, 25, 26 ou 27.

33. Procédé de la revendication 31, dans lequel au moins l'un des anticorps est un anticorps selon la revendication 21.

34. Procédé de la revendication 32, dans lequel un des anticorps est un anticorps selon la revendication 22.

35. Procédé de la revendication 33, dans lequel un des anticorps est un anticorps selon la revendicaiton 23.

36. Procédé de la revendication 30, dans lequel un anticorps est un anticorps monoclonal et l'autre est polyclonal.

37. Procédé de la revendication 35 dans lequel au moins un des anticorps est produit par une lignée cellulaire d'hybridome déposée sous le numéro d'accès ATCC HB10186, ou un de ses mutants qui est capable de produire un anticorps monoclonal réagissant spécifiquement avec M. graminicola.

38. Procédé de la revendication 34, dans lequel au moins un anticorps est produit par la lignée cellulaire d'hybridome déposée sous le numéro d'accès ATCC HB10413, ou un de ses mutants, qui est capable de produire un anticorps monoclonal réagissant spécifiquement avec plus d'une espèce choisie dans le groupe constitué par M. fijiensis, M. fijiensis var. difformis et M. musicola.

39. Procédé de la revendication 34, dans lequel au moins un des anticorps est produit par la lignée cellulaire d'hybridome déposée sous le numéro d'accès ATCC HB10414, ou un de ses mutants, qui est capable de produire un anticorps monoclonal réagissant spécifiquement avec plus d'une espèce choisie dans le groupe consistué par M. fijiensis, M. fijiensis var. difformis, M. graminicola et M. musicola.

40. Composition pour la détection immunologique de la présence ou l'absence de Mycosphaerella dans un échantillon dont on soupçonne qu'il contient des antigènes de Mycosphaerella sous forme d'une trousse de diagnostic comprenant un anticorps lié à un support solide et un anticorps marqué avec une molécule rapporteuse, où les deux anticorps sont capables de réagir avec Mycosphaerella, et au moins un des anticorps est un anticorps monoclonal préparé selon l'une quelconque des revendications 18 à 28.

41. Composition de la revendication 40, dans lequel au moins un anticorps est un anticorps préparé selon l'une quelconque des revendications 18, 20, 21, 23, 24 ou 28.

42. Composition de la revendication 41, dans laquelle au moins un anticorps est un anticorps préparé selon l'une quelconque des revendications 19, 20, 22, 25, 26 ou 27.

43. Composition de la revendication 41, dans laquelle au moins l'un des anticorps est un anticorps selon la revendication 23.

44. Composition de la revendication 43 qui contient l'anticorps produit par la lignée cellulaire d'hybridome déposée sous le numéro d'accès ATCC HB10186, ou un de ses mutants, qui est capable de produire un anticorps monoclonal réagissant spécifiquement avec M. graminicola.

45. Composition de la revendication 42, qui contient l'anticorps produit par la lignée cellulaire d'hybridome déposée sous le numéro d'accès ATCC HB1041 3, ou un de ses mutants, qui est capable de produire un anticorps monoclonal réagissant spécifiquement avec plus d'une espèce choisie dans le groupe constitué par M. fijiensis, M. fijiensis var. difformis et M. musicola.

46. Composition de la revendication 42 qui contient l'anticorps produit par la lignée cellulaire d'hybridome déposée sous le numéro d'accès ATCC HB 10414, ou un de ses mutants, qui est capable de produire un anticorps monoclonal réagissant spécifiquement avec plus d'une espèce choisie dans le groupe constitué par M. fijiensis, M. fijiensis var. difformis, M. graminicola et M. musicola.

47. Composition de l'une quelconque des revendications 45 ou 46, dans laquelle la molécule rapporteuse est une enzyme.

48. Composition de la revendication 47 qui comprend également un substrat pour l'enzyme.

49. Composition de la revendication 41, dans laquelle au moins un anticorps est spécifique de M. fijiensis. 50.- Composition de la revendication 41, dans laquelle au moins un anticorps est spécifique de M. fijiensis var. difformis.
